# EUROPEAN PATENT SPECIFICATION

(11) **EP 4 660 629 B1**
(45) Date of publication and mention of the grant of the patent: **26.08.2026**
(21) Application number: 25174493.4
(22) Date of filing: 06.05.2025
(51) Int. Cl.: G01N 33/566, G01N 33/78

(54) **METHOD FOR DETECTING TRANSTHYRETIN TETRAMER, AND METHOD FOR EVALUATING STABILITY**
VERFAHREN ZUM NACHWEIS VON TRANSTHYRETINTETRAMER UND VERFAHREN ZUR BEURTEILUNG DER STABILITÄT
PROCÉDÉ DE DÉTECTION DE TÉTRAMÈRE DE TRANSTHYRÉTINE, ET PROCÉDÉ D'ÉVALUATION DE LA STABILITÉ

(30) Priority: 31.05.2024 JP 2024089229
(43) Date of publication of application: 10.12.2025
(73) Proprietor: SYSMEX CORPORATION, Kobe-shi, Hyogo 651-0073 (JP); National University Corporation Kobe University, Kobe-shi, Hyogo 657-8501 (JP)
(72) Inventor: IINO, Takuya, Kobe-shi, Hyogo 651-0073 (JP); MURAKAMI, Shun, Kobe-shi, Hyogo 651-0073 (JP); KOBAYASHI, Yuto, Kobe-shi, Hyogo 651-0073 (JP); TOH, Ryuji, Kobe-shi, Hyogo 657-8501 (JP); NAGAO, Manabu, Kobe-shi, Hyogo 657-8501 (JP)
(74) Representative: Hoffmann Eitle

(56) References cited:
- EP-A2- 0 218 309
- WO-A1-2013/074861
- WO-A2-2008/034016
- WO-A2-2011/075210

## Description

### FIELD OF THE INVENTION

The present invention relates to a method for detecting transthyretin (TTR) tetramer. The present invention relates to a method for evaluating stability of TTR tetramer.

### BACKGROUND

TTR monomer is a protein having 127 amino acid residues, and is usually present in the blood in the form of a homotetramer. The TTR tetramer functions as a carrier for transporting thyroxine (also referred to as T4), which is a thyroid hormone, to the cell. The TTR tetramer has two T4 binding sites, and the binding site is formed by binding of two TTR dimers. The TTR tetramer also has a function of transporting retinol via a retinol-binding protein. TTR, formerly also referred to as prealbumin, was named transthyretin because of its transport of thyroxine and retinol.

TTR is a protein rich in β-sheet, and is also known as one of causative proteins of amyloidosis. Amyloidosis caused by TTR is referred to as TTR amyloidosis (ATTR). ATTR includes a type in which mutant TTR accumulates due to a genetic mutation, and a type in which wild-type TTR accumulates, although the cause thereof remains unknown. In any type of ATTR, the TTR tetramer cannot stably maintain its structure, and dissociates into a dimer and a monomer. The dissociated monomer undergoes partial denaturation and misfolding, and aggregates to finally form amyloid fibrils. Amyloid fibers derived from TTR are deposited on the heart, kidney, digestive tract, peripheral nerve, and the like, to cause dysfunction. Although the prognosis of ATTR was poor, the TTR stabilizer tafamidis has recently been developed. Tafamidis binds to the thyroxine binding site to stabilize the TTR tetramer, and inhibits dissociation into the monomer. This suppresses formation of amyloid fibers.

With the development of drugs effective for ATTR such as Tafamidis, it has become important to find ATTR patients and suspected ATTR patients by screening tests and diagnoses. Known screening tests include electrocardiogram, and echocardiography. Diagnostic methods for ATTR include ^{99m} Tc-pyrophosphate scintigraphy, and tissue biopsy. Methods for detecting TTR tetramer in a sample have also been known. Patent Document 1, for example, describes a method for detecting a stable TTR tetramer by adding urea to a sample that contains TTR at a final concentration of 4.8 M, and incubating for three days, thereby removing unstable TTR that is not a tetramer.

### PRIOR ART

### PATENT DOCUMENT

[Patent Document 1] U.S. Patent Application No. 2008/0131907

### SUMMARY OF THE INVENTION

### PROBLEM TO BE SOLVED BY THE INVENTION

Since the underlying cause of ATTR is destabilization of TTR tetramer, a test method capable of evaluating the stability of TTR tetramer in a subject is desired. There are, however, still few methods for detecting TTR tetramer in the sample. It is therefore an object of the present invention to provide a means capable of detecting TTR tetramer in a sample.

### MEANS FOR SOLVING THE PROBLEM

The present inventors have found that TTR tetramer in a sample can be specifically detected with use of a compound capable of binding to the T4 binding site of TTR tetramer, an antibody capable of specifically binding to TTR, and a labeling substance, and have completed the present invention. Hence, the following invention is provided.

A method for detecting TTR tetramer in a sample, the method comprising: forming a complex comprising TTR tetramer in the sample, a compound capable of binding to the T4 binding site of the TTR tetramer, an antibody capable of binding to the TTR monomer, and a labeling substance; and measuring a signal generated by the labeling substance contained in the complex.

A method for evaluating stability of TTR tetramer in a sample, the method comprising: forming a first complex comprising the TTR tetramer in the sample, a compound capable of binding to the T4 binding site of the TTR tetramer, an antibody capable of binding to the TTR monomer, and a labeling substance; and measuring a first signal generated by the labeling substance contained in the first complex, wherein the measured value of the first signal is indicative of stability of the tetramer.

### EFFECT OF THE INVENTION

The present invention relates to a method for detecting TTR tetramer in a sample, a method for evaluating stability of TTR tetramer.

### BRIEF DESCRIPTION OF THE DRAWINGS

[Fig.1A] is a schematic diagram illustrating an exemplary step of forming a complex in the detection method of the present embodiment.
[Fig.1B] is a schematic diagram illustrating an exemplary signal measurement step of the detection method according to the present embodiment.
[Fig.2 A] is a schematic diagram illustrating an exemplary step of forming a complex in the detection method of the present embodiment.
[Fig.2B] is a schematic diagram illustrating an exemplary signal measurement step of the detection method according to the present embodiment.
[Fig.3 A] is a schematic diagram illustrating an exemplary step of forming a complex in the detection method of the present embodiment.
[Fig.3 B] is a schematic diagram illustrating an exemplary signal measurement step of the detection method according to the present embodiment.
[Fig.4 A] is a schematic diagram illustrating an exemplary step of forming a complex in the detection method of the present embodiment.
[Fig.4 B] is a schematic diagram illustrating an exemplary signal measurement step of the detection method according to the present embodiment.
[Fig.5] A reaction scheme illustrating three steps for binding a substance having a carboxy group to a substance having an amino group.
[Fig.6] This is a scheme of a reaction for binding a substance having an N-hydroxysuccinimide (NHS) ester to a substance having an amino group, and a scheme of a reaction for binding a substance having an isothiocyano group to a substance having an amino group.
[Fig.7] This is a scheme of a reaction that bonds a substance having a maleimide group and a substance having a sulfhydryl group, and a scheme of a reaction that bonds a substance having a bromo (or iodo) acetamide group and a substance having a sulfhydryl group.
[Fig.8] is a scheµatic drawing of a reagent according to the present embodiment in a kit form.
[Fig.9 A] is a schematic diagram of a reagent kit according to the present embodiment.
[Fig.9 B] is a schematic diagram of a reagent kit according to the present embodiment.
[Fig. 10 A] is a scheme for a reaction for synthesizing C1 methyl from thyroxine methyl.
[Fig. 10 B] is a scheme of a reaction for synthesizing probe C1 from C1 methyl.
[Fig.11A] is a scheme of a reaction for synthesizing C2 methyl from thyroxine methyl.
[Fig.11B] is a scheme of a reaction for synthesizing probe C2 from C2 methyl.
[Fig. 12 A] is a scheme of a reaction for synthesizing Tafaµidis-ACl from Tafamidis.
[Fig. 12 B] Scheme of Reaction for Synthesizing Probe C3 from Tafamidis-ACl.
[Fig.12 C] Scheme of Reaction for Synthesizing Probe C4 from Tafamidis-ACl.
[Fig. 13 A] is a scheme of a reaction for synthesizing (E)-4-(4-aminostyryl)-2,6-dibromo-4-(4-nitrostyryl)phenol from (E)-2,6-dibromo-4-nitrostyryl)phenol.
[Fig. 13 B] is a scheme of a reaction for synthesizing probe C5 from (E)-4-(4-aminostyryl)-2,6-dibromophenol.
[Fig.13 C] is a scheme of a reaction for synthesizing probe C6 from (E)-4-(4-aminostyryl)-2,6-dibromophenol.
[Fig. 14 A] is a graph illustrating measured results of a sample that contains the recombinant TTR of Example 1.
[Fig. 14 B] is a graph illustrating measured results of a sample that contains the recombinant TTR of Example 1.
[Fig.14 C] is a graph illustrating measured results of a sample that contains the recombinant TTR of Example 1.
[Fig. 14 D] is a graph illustrating measured results of a sample that contains the recombinant TTR of Example 1.
[Fig.15 A] is a graph illustrating measured results of a serum sample of Example 1.
[Fig. 15 B] is a graph illustrating measured results of a plasma sample of Example 1.
[Fig.16 A] This is a graph illustrating measured results of a sample that contains the recombinant TTR of Example 2.
[Fig.16 B] is a graph illustrating measured results of a sample that contains the recombinant TTR of Example 2.
[Fig.17 A] is a graph illustrating measured results of a serum sample of Example 2.
[Fig.17 B] is a graph illustrating measured results of a plasma sample of Example 2.
[Fig.17 C] is a graph illustrating measured results of a serum sample of Example 2.
[Fig.17 D] is a graph illustrating measured results of a plasma sample of Example 2.
[Fig.18 A] This is a graph illustrating measured results of a sample that contains the recombinant TTR of Example 3.
[Fig.18 B] is a graph illustrating measured results of a sample that contains the recombinant TTR of Example 3.
[Fig.19 A] is a graph illustrating measured results of a plasma sample of Example 3.
[Fig.19 B] is a graph illustrating measured results of a plasma sample of Example 3.
[Fig.20 A] This is a graph illustrating a result of measuring a sample that contains the recombinant TTR of Example 4, with use of the detection method of this embodiment.
[Fig.20 B] This is a graph illustrating a result of measuring, with a sandwich ELISA, a sample that contains the recombinant TTR of Example 4.
[Fig.20 C] This is a graph illustrating corrected values for the amount of TTR tetramer, for a sample that contains the recombinant TTR of Example 4.
[Fig.21 A] This is a graph illustrating results of measuring a sample that contains the recombinant TTR of Example 5, with an fully automated immunoassay system.
[Fig.21 B] This is a graph illustrating a result of measuring a sample that contains the recombinant TTR of Example 5, with an fully automated immunoassay system.
[Fig.22 A] This is a graph illustrating a result of measuring a sample that contains the recombinant TTR of Example 6, with an fully automated immunoassay system.
[Fig.22 B] This is a graph illustrating results of measuring a sample that contains the recombinant TTR of Example 6, with an fully automated immunoassay system.
[Fig.23 A] This is a graph illustrating a result of measuring a sample that contains the recombinant TTR of Comparative Example 1, with sandwich ELISA.
[Fig.23 B] This is a graph illustrating a result of measuring a serum sample of Comparative Example 1 with sandwich ELISA.
[Fig.24 A] This is a graph illustrating results of a sample that contains the recombinant TTR of Comparative Example 2, treated with urea for 48 hours, and then measured by sandwich ELISA.
[Fig.24 B] This is a graph illustrating a result of measuring the serum sample of Comparative Example 2 by sandwich ELISA, after being urea-treated for 48 hours.
[Fig.25] This is a graph illustrating results of measuring a sample that contains the recombinant TTR of Comparative Example 3, with use of a sandwich ELISA, after being urea-treated for one hour.

### DETAILED DESCRIPTION OF THE PREFERRED EMBODIMENTS

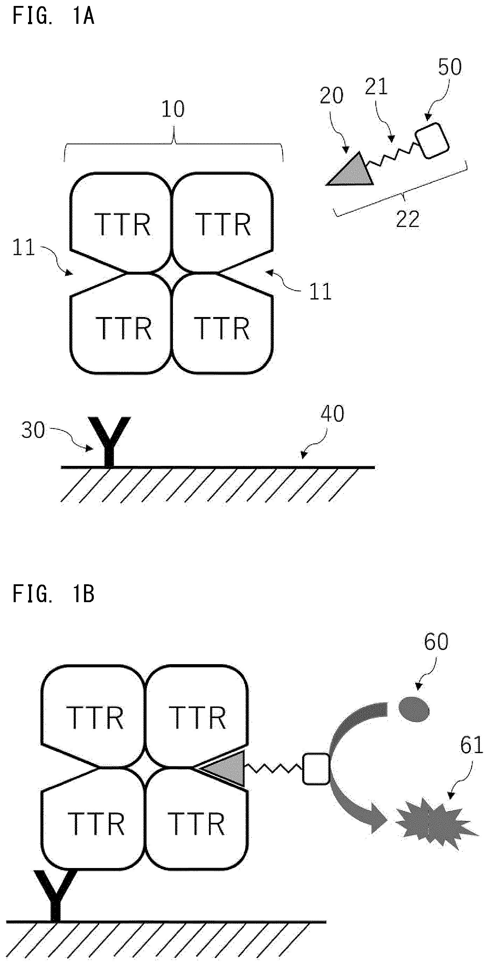

The method for detecting TTR tetramer in the sample of the present embodiment (hereinafter, also referred to as "detection method of the present embodiment") is a method for detecting TTR tetramer contained in a sample in vitro. In the detection method of the present embodiment, after forming the complex, a signal generated by the labeling substance contained in the complex is measured. Referring to Figs.1A and B, an exemplary process of forming the complex and a process of measuring the signal will be described. Fig.1A represents a state before the complex is formed in the complex forming step. Fig.1B shows a state where a signal is generated by a labeling substance in the complex in the signal measurement step.

The complex is formed by mixing a sample that contains TTR, a compound capable of binding to the T4 binding site of the TTR tetramer (also referred to as "ligand"), an antibody capable of binding to the TTR monomer (also referred to as "anti-TTR antibody"), and a labeling substance. The order in which they are mixed is not specially limited. The complex is usually formed in a liquid. In this specification, the term "comprising TTR" is intended to include any or all of TTR monomer, TTR dimer, and TTR tetramer. In a sample that contains TTR, the TTR dimer and the TTR tetramer are usually naturally formed. As illustrated in Fig.1A, there are two T4 binding sites (11) in one TTR tetramer (10). One molecule of ligand is bound to one T4 binding site. In forming the complex, one molecule of the ligand may bind to any one of the two T4 binding sites of the TTR tetramer. Alternatively, two molecules of ligand may bind to each of the two T4 binding sites of the TTR tetramer. The anti-TTR antibody binds to any of four TTR monomers constituting the TTR tetramer. That is, the anti-TTR antibody binds to the TTR tetramer. The labeling substance may indirectly bind to the TTR tetramer via a ligand bound to the T4 binding site. Alternatively, the labeling substance may indirectly bind to the TTR tetramer via an anti-TTR antibody bound to the TTR tetramer. In the example of Fig.1A, the labeling probe (22) is used as the ligand that contains the labeling substance. In the labeled probe (22), the ligand (20) and the labeling substance (50) are covalently bonded to each other via a linker (21). The labeled probe will be described later. Referring to Fig.1B, the labeling substance (50) may indirectly bind to the TTR tetramer (10) by binding of the ligand (20) in the labeling probe (22) to the T4 binding site (11).

The complex is preferably formed on the solid phase. The complex is usually formed on the solid phase in a liquid. In the example of Fig.1A, the anti-TTR antibody (30) has been immobilized on the solid phase (40) in advance. The anti-TTR antibody, however, is not limited thereto, and may be immobilized on the solid phase during or after formation of the complex. The term "forming the complex" means a state in which any of the anti-TTR antibody, ligand, and labeling substance have not yet bound to the TTR tetramer. Referring to Fig.1B, anti-TTR antibody (30) binds to TTR tetramer (10) on solid phase (40). Also, as described previously, the ligand (20) in the labeled probe (22) binds to the T4 binding site (11), so that the labeling substance (50) indirectly binds to the TTR tetramer (10). This forms a complex that contains the TTR tetramer (10), the ligand (20), the anti-TTR antibody (30), and the labeling substance (50). The anti-TTR antibody (30) serves as a capture body for the TTR tetramer, and "capture body" refers to a substance that specifically binds to a test substance, and is immobilized on a solid phase. The test substance is captured on the solid phase, by binding of the capture body and the test substance. The capture body may be immobilized on the solid phase in advance. In the example of Fig. 1B, the labeling substance (50) is an enzyme. Signal (61) is generated by reacting substrate (60) with labeling substance (50), which is an enzyme. Referring to Fig.1B, in the detection method of the present embodiment, the TTR tetramer (10) in the sample may be detected by measuring the signal (61). Samples, reagents, and the like used in the detection method of the present embodiment will be described below.

The sample is not particularly limited as long as it contains TTR. The sample is preferably a biological sample collected from a subject. The biological sample is exemplified by blood sample and cerebrospinal fluid. The blood sample is exemplified by blood (whole blood), plasma, and serum. The preferred sample is plasma or serum. The subject is not specially limited, and is exemplified by healthy persons, ATTR patients, and suspected ATTR. Any insoluble impurity such as cell, if contained in the sample, may be removed therefrom by a known technique such as centrifugation or filtration. The sample may optionally be diluted with an appropriate aqueous solvent. Such aqueous solvent is exemplified by water, saline and buffer. The buffer is exemplified by phosphate-buffered saline (PBS), Tris-HCl and Good's buffer.

In the detection method of the present embodiment, the ligand and the anti-TTR antibody are used as the capture body and the detector of the TTR tetramer. The capture body is as described above, and the "detector" refers to a substance that specifically binds to the test substance, and provides a signal detectable through the labeling substance. The detector is usually not immobilized on the solid phase. The detector preferably contains a labeling substance. In the detection method of the present embodiment, the ligand may be used as the capture body, and the anti-TTR antibody may be used as the detection body. Alternatively, the ligand may be used as the detector, using the anti-TTR antibody as the capture body.

The ligand is selected from the low-molecular-weight compounds capable of stabilizing the tetramer structure of TTR tetramer by entering into and binding to the T4 binding site of TTR tetramer. Such low-molecular-weight compound per se has been known, and is referred to as TTR stabilizer, TTR kinetic stabilizer, etc. The ligand preferably has a functional group and/or a substituent that interacts with amino acid residue constituting the T4 binding site in the TTR tetramer. Examples of such functional group and substituent include carboxy group, hydroxyl group, methyl group, halogenated methyl group, amino group and halogen atom. The halogen atom is fluorine, chlorine, bromine or iodine. The ligand may bind to the T4 binding site of the TTR tetramer, by interacting with the functional group and/or substituent of the ligand, and the amino acid residue constituting the T4 binding site. The binding mode of the ligand and the T4 binding site is not specially limited. This is exemplified by hydrophobic interaction, electrostatic interaction, hydrogen bond, and combinations thereof. Among the amino acid residues constituting the T4 binding site, known amino acid residues interact with ligands, such as Lys15,Leu17,Glu54,Ser 117 and Thr119.

The binding of the TTR tetramer in the sample to the ligand is exemplified by mixing a sample that contains TTR, and the ligand. The mixture that contains the sample and the ligand preferably incubates at a temperature of 4° C. or higher and 40° C. or lower, for example. The incubation time may be properly determined, without special limitation. For example, when the mixture containing the sample and the ligand is incubated at room temperature (15° C. or higher and 30° C. or lower), the incubation time may be 1 minute or longer and 3 hours or shorter. The mixture may rest, stir or shake during incubation.

In this specification, the term "antibody" encompasses a full-length antibody and a fragment thereof. The fragments of the antibody are exemplified by Fab,Fab',F(ab')2, Fd, Fd', Fv, light chain, heavy chain variable region (VHH) of the heavy chain antibody, reduced IgG (rIgG), and single chain antibody (scFv). The antibody may be either monoclonal antibody or polyclonal antibody. The antibody may be derived from any mammal such as mouse, rat, hamster, rabbit, goat, horse, or camel, without special limitation.

The anti-TTR antibody preferably binds to a site other than a site not exposed on the surface, when the TTR tetramer is formed, in the TTR monomer. The anti-TTR antibody that binds to such site of the TTR monomer may not bind to the TTR tetramer. The anti-TTR antibody is therefore preferably an antibody capable of binding to both the TTR monomer and the TTR tetramer. The anti-TTR antibody preferably binds to a site other than the site that constitutes the T4 binding site, in the TTR monomer. This is for avoiding competition with the ligand in binding of the anti-TTR antibody and the TTR tetramer. The anti-TTR antibody per se has been known, and a commercially available anti-TTR antibody may be used. The commercially available anti-TTR antibody is exemplified by polyclonal rabbit anti-human prealbumin antibody (Agilent Corporation, A0002), and monoclonal mouse anti-human prealbumin antibody (Medix Biochemica Corporation, 100828, clone 11601).

The binding of the TTR tetramer in the sample to the anti-TTR antibody is exemplified by mixing a sample that contains TTR, and an anti-TTR antibody. The mixture that contains the sample and the anti-TTR antibody preferably incubates at a temperature of 4° C. or higher and 40° C. or lower, for example. The incubation time may be properly determined, without special limitation. For an exemplary case where the mixture containing the sample and the anti-TTR antibody is incubated at 15° C. or higher and 40° C. or lower, the incubation time may be 1 minute or longer and 3 hours or shorter. The mixture may rest, stir or shake during incubation.

When the anti-TTR antibody is used as the detector, the anti-TTR antibody preferably contains a labeling substance. When the anti-TTR antibody contains a labeling substance, the anti-TTR antibody and the labeling substance may directly bind, for example. Alternatively, the anti-TTR antibody and the labeling substance may be indirectly bound while placing some other substance in between. The direct binding of the anti-TTR antibody to the labeling substance is exemplified by covalently binding the anti-TTR antibody to the labeling substance, using a commercially available crosslinker or labeling kit. The antibody having the labeling substance bound thereto by a covalent bond is also referred to as "labeled antibody". The anti-TTR antibody having the labeling substance bound thereto by a covalent bond is also referred to as "labeled anti-TTR antibody". The indirect binding of the anti-TTR antibody and the labeling substance is exemplified by binding of the anti-TTR antibody to a labeled antibody (labeled secondary antibody) that specifically binds to the anti-TTR antibody.

The labeling substance refers to a substance that provides a detectable signal by itself or via contact with other substance. The labeling substance is exemplified by substance that catalyzes reaction of some other substance to generate the signal, and substance that generates the signal by itself (hereinafter, also referred to as "signal generating substance"). The substance that catalyzes reaction of some other substance to generate the signal is exemplified by enzyme. The signal generating substance is exemplified by fluorescent substance, compound containing radioisotope, and chemiluminescent substance. The enzyme is exemplified by alkaline phosphatase (ALP), peroxidase (POD), β-galactosidase, and luciferase. The fluorescent substance is exemplified by fluorescent dyes such as fluorescein isothiocyanate (FITC), rhodamine, and Alexa Fluor (registered trademark); fluorescent protein such as green fluorescent protein (GFP), and yellow fluorescent protein (YFP). The compound that contains a radioisotope is exemplified by nucleic acids that contain any of ¹²⁵ I, ¹⁴ C, ³² P, ^{99m} Tc, ²²⁵ Ac, saccharide, and oligopeptide. The chemiluminescent substance is exemplified by ruthenium pyridine complex and acridinium ester. The labeling substance is preferably enzyme, and ALP and POD are particularly preferred. The labeling substance may further contain a spacer arm having a reactive group at its end, so as to bind to the detector. The reactive group refers to a group that selectively reacts with a predetermined functional group. The reactive group may properly be determined depending on the functional group of the detector. The reactive group is exemplified by NHS ester and maleimide group. The spacer arm is exemplified by a linear saturated aliphatic hydrocarbon chain, and a polyethylene glycol (PEG) chain.

In a case where the labeling substance is an enzyme, the detection method of the present embodiment uses the substrate of the enzyme in signal measurement. The substrate may properly be selected from known substrates, depending on the type of enzyme. For example, when ALP is used as the enzyme, the substrate is exemplified by chemiluminescent substrates such as CDP-Star (registered trademark) (disodium 4-chloro-3-(methoxyspiro[1,2-dioxetane-3,2'-(5'-chloro)tricyclo[3.3.1.13,7]decane]-4-yl)phenylphosphate) and CSPD (registered trademark) (disodium 3-(4-methoxyspiro[1,2-dioxetane-3,2-(5'-chloro)tricyclo[3.3.1.13,7]decane]-4-yl)phenylphosphate). Another substrate includes chromogenic substrates such as 5-bromo-4-chloro-3-indolylphosphate (BCIP), disodium 5-bromo-6-chloro-indolylphosphate, and p-nitrophenyl phosphate. In an exemplary case where POD is used as the enzyme, the substrate is exemplified by chemiluminescent substrates such as luminol and derivatives thereof; and chromogenic substrates such as ammonium 2,2'-azinobis(3-ethylbenzothiazoline-6-sulfonate) (ABTS), 1,2-phenylenediamine (OPD), and 3,3',5,5'-tetramethylbenzidine (TMB). In a preferred embodiment, the signal is a chemiluminescent signal generated by contacting the enzyme with a substrate.

The solid phase may only be an insoluble carrier on which the capture body may be immobilized. Material for composing the solid phase is selectable, without special limitation, typically from organic polymer compound, inorganic compound, and biopolymer. The organic polymer compound is exemplified by latex, polystyrene and polypropylene. The inorganic compound is exemplified by magnetic substance (iron oxide, chromium oxide, ferrite, etc.), silica, alumina and glass. The biopolymer is exemplified by insoluble agarose, insoluble dextran, gelatin and cellulose. Two or more of them may be used in combination. The solid phase may have any form not specifically limited, and is exemplified by particle, microplate, microtube, test tube and membrane. Among them, particle (particularly magnetic particle) and microplate are preferred. The solid phase may further contain a spacer arm having a reactive group at its end, for example, for binding to the capturing agent.

Mode of immobilization of the anti-TTR antibody on the solid phase is not specially limited, unless the anti-TTR antibody is used as the capture body. For example, the anti-TTR antibody and the solid phase may be directly bound, or the anti-TTR antibody and the solid phase may be indirectly bound while placing some other substance in between. The direct binding of the solid phase and the antibody is exemplified by adsorption on the surface of the solid phase by hydrophobic interaction, or covalent binding. For an exemplary case where the solid phase is a microplate for ELISA, the antibody is immobilized in the well of the plate by adsorption. In an exemplary case where the solid phase has a functional group on the surface, the antibody may be immobilized on the surface of the solid phase by covalent bonding utilizing the functional group. In an exemplary case where the solid phase is a particle having a carboxy group, the carboxy group on the particle surface is activated with 1-[3-(dimethylamino)propyl]-3-ethylcarbodiimide (WSC), and then reacted with NHS to form an NHS ester. Then, when the particle having NHS ester is contacted with the antibody, the NHS ester reacts with the amino group of the antibody, and the antibody is immobilized on the particle surface by a covalent bond.

The indirect binding between the solid phase and the antibody is exemplified by binding through a molecule capable of specifically binding to the antibody. The antibody may be immobilized on the solid phase, by preliminarily immobilizing such molecule on the surface of the solid phase. The molecule that specifically binds to the antibody is exemplified by protein A and protein G. Alternatively, the antibody and the solid phase may be bound by using a combination of the tag and a binding partner that specifically binds to the tag (also referred to as "binding partner"). The tag is not specially limited as long as it is a substance different from the labeling substance, and its binding partner is present. The anti-TTR antibody may be immobilized on the solid phase, via a bond between the tag and the binding partner, by adding a tag to the anti-TTR antibody, and preliminarily immobilizing the binding partner on the solid phase.

The combination of the tag and the binding partner per se has been known, and is exemplified by combinations of biotins and avidins, hapten and anti-hapten antibody, glutathione-S-transferase (GST), glutathione, histidine tag (a peptide that contains histidine of 6 to 10 residues), and Ni-NTA (nitrilotriacetic acid having a chelate with nickel ion). In this specification, the term "biotins" encompasses biotin and biotin analog. The biotin analog is exemplified by desthiobiotin and biocytin. In this specification, the term "avidins" encompasses avidin and avidin analog. The avidin analog is exemplified by streptavidin, avidin-like protein derived from Paecilomyces tenuipes (Tamavidin (registered trademark)), bradavidin, and rhizavidin. The hapten is, for example, a 2,4-dinitrophenyl (DNP) hapten, and the binding partner is an anti-DNP antibody. The tag is preferably biotins, and biotin is particularly preferred. The binding partner is preferably avidins, and streptavidin is particularly preferred. The tag may further have a spacer arm having a reactive group at its terminal, for example, so as to bind to the capture body or the detector.

When the ligand is used as the detector, the ligand preferably contains a labeling substance. In an exemplary case where the ligand contains a labeling substance, the ligand is previously covalently bonded to the labeling substance, either directly or via a linker. In this specification, a ligand having a labeling substance covalently bonded thereto directly or via a linker is also referred to as "labeled probe". Alternatively, the ligand may comprise a tag, and the labeling substance may comprise a binding partner. In an exemplary case where the ligand contains a tag, the ligand is previously covalently bonded to the tag either directly or via a linker. In this specification, a ligand having a tag covalently bonded thereto directly or via a linker is also referred to as "tagged probe". In an exemplary case where the labeling substance contains a binding partner, the labeling substance is previously covalently bonded to the binding partner directly or via a linker. The ligand and the labeling substance may be indirectly bound via a bond between the tag and the binding partner, since the tag is previously bound to the ligand, and the binding partner is previously bound to the labeling substance. Commercially available reagents such as streptavidin-horseradish peroxidase (HRP), and streptavidin-ALP may be used as the labeling substance, to which the binding partner is covalently bonded directly or via a linker.

In an exemplary case where the ligand is used as the capture body, the ligand preferably has been immobilized on the solid phase in advance. For example, the ligand is previously covalently bonded to the solid phase, either directly or via a linker. Alternatively, the ligand may comprise a tag, and the solid phase may comprise a binding partner. That is, the capture body is a tagged probe, and the solid phase is previously covalently bonded to the binding partner, either directly or via a linker. The ligand and the solid phase may be indirectly bound via a bond between the tag and the binding partner, by preliminarily binding the tag to the ligand, and preliminarily binding partner to the solid phase.

The T4 binding site is known to be a cavity also referred to as a T4 binding pocket. The ligand enters the cavity, and interacts with the amino acid residue that constitutes the T4 binding site. The ligand may therefore enter the T4 binding site, preferably by covalently binding the ligand, the tag, the labeling substance, or the solid phase, via a linker, and the tag or the like may be exposed outside the T4 binding site.

The ligand having the tag, the labeling substance, or the solid phase covalently bonded thereto via a linker may be, for example, a compound represented by formula (I) below. In Formulae (I), the moiety represented by "-X ¹ -L-X ²-" corresponds to a linker that connects the ligand and the tag, the labeling substance, or the solid phase. The length of the moiety represented by "-X ¹ -L ² -X ²-" may be, for example, about 5 Å or more and about 95 Å or less. Hereinafter, substituents of Formula (I) and the like will be described.

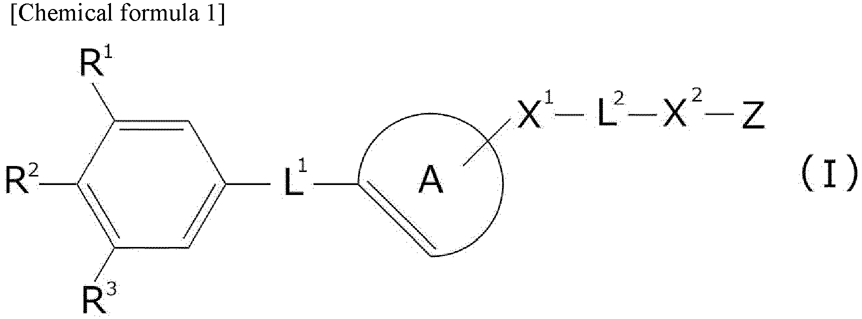

In Formula (I), R ¹ and R ³ are the same or different and each represents a halogen atom, a methyl group or a halogenated methyl group. R ² represents a hydrogen-atom, a hydroxyl group or an amino-group. L ¹ is a bond, an oxygen-atom, a sulfur-atom, or - CH=CH-, -CH ₂ -CH ₂-, -N=N-, or - (C=O)-. 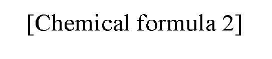 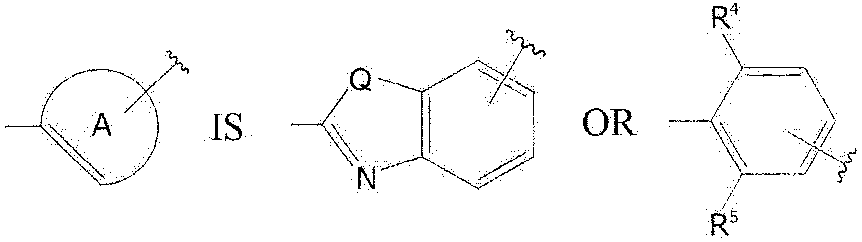 Q is an oxygen atom, a sulfur atom, or -NH-. R ⁴ and R ⁵ are the same or different and represent a hydrogen-atom or a halogen-atom. X ¹ and X ² are the same or different and represent -R⁶-NH-, -NH-R⁶-, -R⁶-(C=O)-NH-, -(C=O)-NH-R⁶-, -R⁶-NH-(C=O)-, -NH-(C=O)-R⁶-, -R⁶-(C=O)-, -(C=O)-R⁶-, -R⁶-(C=O)-O-, -(C=O)-O-R⁶-, -R⁶-O-(C=O)-, -O-(C=O)-R⁶-, -R⁶-(C=S)-NH-, -(C=S)-NH-R⁶-, -R⁶-NH-(C=S)-, -NH-(C=S)-R⁶-, -R⁶-O-, - O-R⁶-, -R⁶-S- or -S-R⁶-.

R ⁶ each independently represents a bond, an alkylene group having 1 to 10 carbon atoms which may have a substituent, an arylene group having 6 to 12 carbon atoms which may have a substituent, a heteroarylene group having 4 to 12 carbon atoms which may have a substituent, a cycloalkylene group having 3 to 8 carbon atoms which may have a substituent, or a heterocycloalkylene group having 2 to 8 carbon atoms which may have a substituent. L ² is represented by -(CH ₂) ₐ -[X ³ - (CH ₂) _{b}] _{c} - or -[(CH ₂) _{b} - X ³] _{c} (CH ₂) ₐ -. X ³ is an oxygen-atom, a sulfur-atom, or -NH-, -NH-(C=O)-, -(C=O)-NH-, or a bond. a and b are the same or different and are integers equal to or greater than 1 and equal to or less than 6, c is an integer of 1 or more and 24 or less, and. Z comprises a tag, a labeling substance, or a solid phase.

In this specification, the term "atomic bond" refers to a direct bond without intervening any other atom. In Formula (I), when R ⁶ represents an alkylene group having 1 to 10 carbon atoms, examples of the alkylene group include methylene, ethylene, propylene, isopropylene, butylene, isobutylene, pentylene, neopentylene, hexylene, heptylene, octylene, 2-ethylhexylene, nonylene, and decylene. Among them, an alkylene group having 1 to 4 carbon atoms is preferred. When R ⁶ represents an alkylene group having a substituent, the number of carbon atoms described above does not include the number of carbon atoms of the substituent.

When R ⁶ is an arylene group having 6 to 12 carbon atoms, such group is exemplified by phenylene, naphthylene and biphenylene. When R ⁶ is a heteroarylene group having 4 to 12 carbon atoms, such group may only be an aromatic ring having 4 to 12 carbon atoms which contains one or more heteroatoms selected from N, S, O and P. This is exemplified by groups such as furanylene, pyrrolene, thiophenylene, triazolene, oxadiazolene, pyridylene and pyrimidylene. When R ⁶ represents an arylene group or a heteroarylene group having a substituent, the above carbon number does not include the carbon number of the substituent.

When R ⁶ is a cycloalkylene group having 3 to 8 carbon atoms, such group is exemplified by cyclopropylene, cyclobutylene, cyclopentylene, cyclohexylene, cycloheptylene and cyclooctylene. When R ⁶ is a heterocycloalkylene group having 2 to 8 carbon atoms, such group may only be a non-aromatic ring having 2 to 8 carbon atoms that contains one or more heteroatoms selected from N, S, O and P. This is exemplified by groups such as oxacyclopropylene, oxazolidinylene, pyrrolidinylene, piperidinylene and morpholinylene. When R ⁶ represents a cycloalkylene group or a heterocycloalkylene group having a substituent, the number of carbon atoms described above does not include the number of carbon atoms of the substituent.

The substituent in the R ⁶ is exemplified by groups such as carboxy, cyano, alkoxy, nitro, =O, =S, -SH, halogen-atom, haloalkyl, heteroalkyl, carboxyalkyl, amine, amido and thioether. R ⁶ and R ⁷ may have a plurality of substituents. The halogen atom is fluorine, chlorine, bromine or iodine. The alkoxy represents an -O-alkyl group, and the alkyl group is a linear or branched saturated aliphatic hydrocarbon group having 1 to 5 carbon atoms, preferably 1 or 2 carbon atoms.

In Formula (I), the L ² corresponds to a spacer arm, and has a linear conformation imparting a predetermined length to the linker. X ¹ corresponds to a linking moiety between the ligand and L ², and X ² corresponds to a linking moiety between L ² and Z. Z is preferably a tag, a labeling substance, or a solid phase. The L ² preferably contains a hydrophilic polymer. In Formulae (I), X ³ preferably represents an oxygen-atom. L ² at this time is represented by -(CH ₂) ₐ -[O-(CH ₂) _{b}] _{c} - or -[(CH ₂) _{b} - O] _{c} (CH ₂) ₐ -. a and b are the same or different and are integers equal to or greater than 1 and equal to or less than 6, and c is an integer equal to or greater than 1 and equal to or less than 24. Preferably a and b are the same or different and are integers equal to or greater than 1 and equal to or less than 4. a and b are more preferably 2. When a and b are 2, L ² is a PEG-chain. The lower limit of c is preferably 2, and more preferably 3. The upper limit of c is preferably 23, and more preferably 22. The PEG chain has been known to have a (PEG) ₄ of about 15 Å, a (PEG) ₈ of about 31 Å, a (PEG) ₁₂ of about 45 Å, and a (PEG) ₂₄ of about 88 Å, for example. The compound represented by formula (I), wherein L ² is a PEG-chain, is exemplified by compounds represented by formula (II), (III), or (IV) below. R ⁶ in the X ² of these formulas are defined as in formula (I). 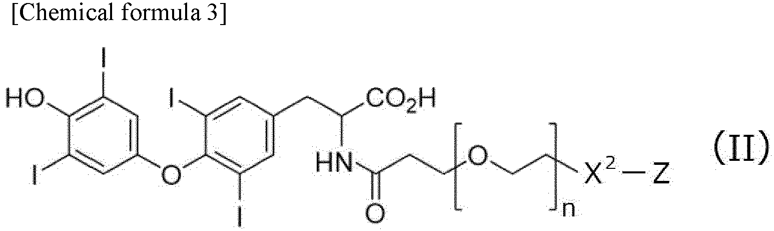
wherein Z comprises a labeling substance or a solid phase, and n is an integer of 1 or more and 24 or less,
X² represents -R⁶-NH-, -NH-R⁶-, -R⁶-(C=O)-NH-, -(C=O)-NH-R⁶-, -R⁶-NH-(C=O)-, - NH-(C=O)-R⁶-, -R⁶-(C=O)-, -(C=O)-R⁶-, -R⁶-(C=O)-O-, -(C=O)-O-R⁶-, -R⁶-O-(C=O)-, -O-(C=O)-R⁶-, -R⁶-(C=S)-NH-, -(C=S)-NH-R⁶-, -R⁶-NH-(C=S)-, -NH-(C=S)-R⁶-, -R⁶-O-, -O-R⁶-, -R⁶-S- or -S-R⁶-,
R ⁶ each independently represents a bond, an alkylene group having 1 to 10 carbon atoms which may have a substituent, an arylene group having 6 to 12 carbon atoms which may have a substituent, a heteroarylene group having 4 to 12 carbon atoms which may have a substituent, a cycloalkylene group having 3 to 8 carbon atoms which may have a substituent, or a heterocycloalkylene group having 2 to 8 carbon atoms which may have a substituent.

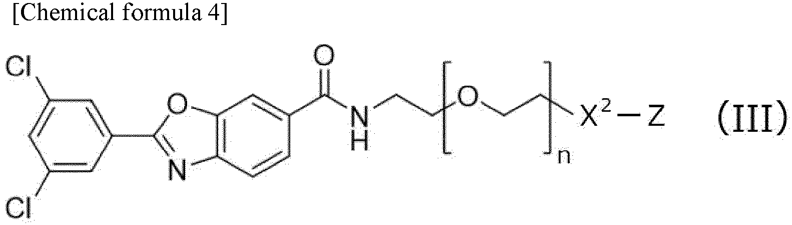

wherein Z comprises a labeling substance or a solid phase, and n is an integer of 1 or more and 24 or less,
X² represents -R⁶-NH-, -NH-R⁶-, -R⁶-(C=O)-NH-, -(C=O)-NH-R⁶-, -R⁶-NH-(C=O)-, - NH-(C=O)-R⁶-, -R⁶-(C=O)-, -(C=O)-R⁶-, -R⁶-(C=O)-O-, -(C=O)-O-R⁶-, -R⁶-O-(C=O)-, -O-(C=O)-R⁶-, -R⁶-(C=S)-NH-, -(C=S)-NH-R⁶-, -R⁶-NH-(C=S)-, -NH-(C=S)-R⁶-, -R⁶-O-, -O-R⁶-, -R⁶-S- or -S-R⁶-,
R⁶ each independently represents a bond, an alkylene group having 1 to 10 carbon atoms which may have a substituent, an arylene group having 6 to 12 carbon atoms which may have a substituent, a heteroarylene group having 4 to 12 carbon atoms which may have a substituent, a cycloalkylene group having 3 to 8 carbon atoms which may have a substituent, or a heterocycloalkylene group having 2 to 8 carbon atoms which may have a substituent.

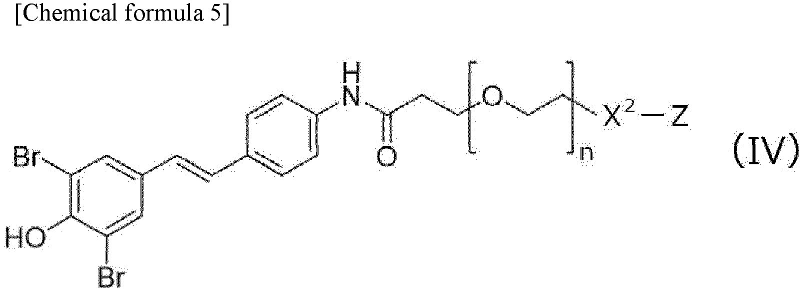

wherein Z comprises a labeling substance or a solid phase, and n is an integer of 1 or more and 24 or less,
X² represents -R⁶-NH-, -NH-R⁶-, -R⁶-(C=O)-NH-, -(C=O)-NH-R⁶-, -R⁶-NH-(C=O)-, - NH-(C=O)-R⁶-, -R⁶-(C=O)-, -(C=O)-R⁶-, -R⁶-(C=O)-O-, -(C=O)-O-R⁶-, -R⁶-O-(C=O)-, -O-(C=O)-R⁶-, -R⁶-(C=S)-NH-, -(C=S)-NH-R⁶-, -R⁶-NH-(C=S)-, -NH-(C=S)-R⁶-, -R⁶-O-, -O-R⁶-, -R⁶-S- or -S-R⁶-,
R ⁶ each independently represents a bond, an alkylene group having 1 to 10 carbon atoms which may have a substituent, an arylene group having 6 to 12 carbon atoms which may have a substituent, a heteroarylene group having 4 to 12 carbon atoms which may have a substituent, a cycloalkylene group having 3 to 8 carbon atoms which may have a substituent, or a heterocycloalkylene group having 2 to 8 carbon atoms which may have a substituent.

In Formula (I), X ¹ preferably represents -R ⁶-(C=O)-NH-, or -R ⁶ -NH-(C=O)-. X ² preferably represents -NH-(C=O)-R ⁶-, or -(C=O)-NH-R ⁶-. In this case, R ⁶ preferably each independently represents an alkylene group having 1 to 6 carbon atoms which does not have a substituent, or an alkylene group having 1 to 6 carbon atoms which has a carboxy group as a substituent.

When the compound represented by formula (I) is a tagged probe, Z in formula (I) preferably represents a biotin group. In this specification, the "biotin group" refers to a heterocyclic moiety that contains at least an imidazolidine ring, among chemical structures of biotins. The preferred biotin group is the biotin group of biotin. The compound represented by formula (I) wherein Z is a biotin group is exemplified by compounds represented by formulae (V), (VI) and (VII) below. 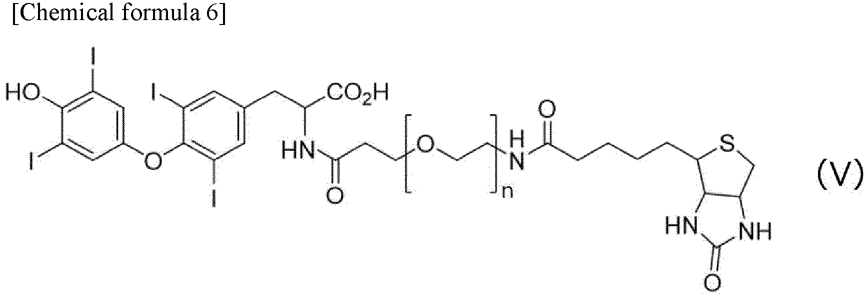 Wherein n is an integer of 1 or more and 24 or less, 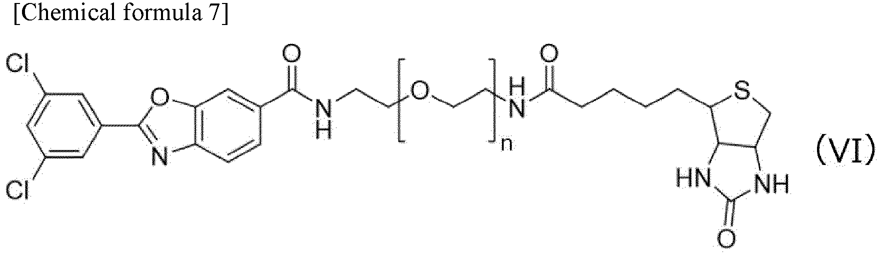 Wherein n is an integer of 1 or more and 24 or less, 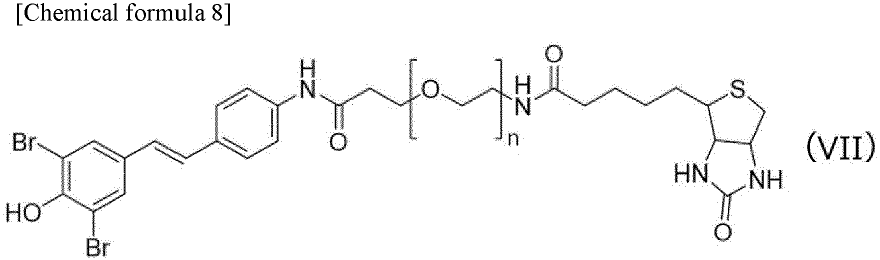 Wherein n is an integer of 1 or more and 24 or less,

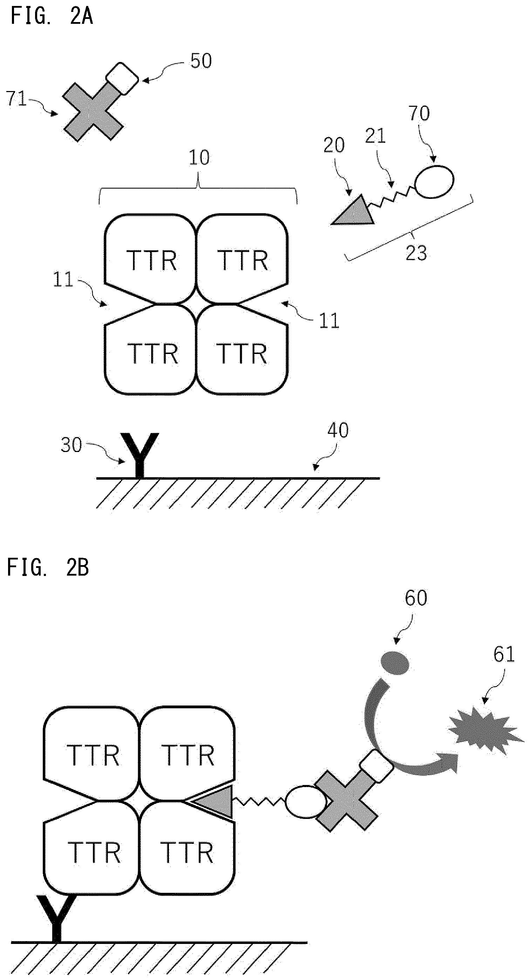

Examples of the detection method of the present embodiment, wherein the TTR tetramer is detected with use of the labeled probe and the capture body (anti-TTR antibody), have been described above with reference to Figs. 1A and B. In yet another example of the detection method of the present embodiment, an example of detecting the TTR tetramer with use of the tagged probe, a labeling substance that contains the binding partner, and the capture body (anti-TTR antibody) will be described with reference to Figs.2A and B. The invention, however, is not limited thereto. Fig.2A represents a state before the complex is formed in the complex forming step. Fig.2B shows a state where a signal is generated by a labeling substance in the complex in the signal measurement step. In the example of Fig.2A, the tagged probe (23) is used as the detector. In the tagged probe (23), the ligand (20) and the tag (70) are covalently bonded to each other via a linker (21). Also, the binding partner (71) is directly covalently bonded to the labeling substance (50). The anti-TTR antibody (30) as the capture body has been immobilized on the solid phase (40) in advance. The anti-TTR antibody may, however, be immobilized on the solid phase during or after formation of the complex, without being limited to this example.

Referring to Fig.2B, the ligand (20) in the tagged probe (23) binds to the T4 binding site (11). Further, the labeling substance (50) is added to the tagged probe (23) by binding of the tag (70) in the tagged probe (23) and the binding partner (71). Alternatively, after the labeling substance (50) is added to the tagged probe (23), the ligand (20) in the tagged probe (23) may bind to the T4 binding site (11). This enables the labeling substance (50) to indirectly bind to the TTR tetramer (10). Anti-TTR antibody (30) also binds to TTR tetramer (10) on solid phase (40). This forms a complex that contains the TTR tetramer (10), the ligand (20), the anti-TTR antibody (30), and the labeling substance (50). In Fig.2B, the labeling substance (50) is an enzyme. Signal (61) is generated by reacting substrate (60) with labeling substance (50), which is an enzyme. The TTR tetramer (10) in the sample may be detected by measuring the signal (61).

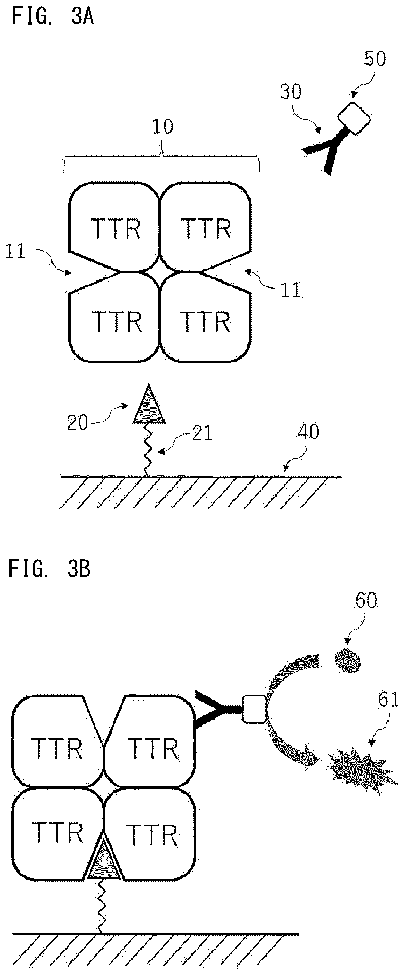

When the ligand is used as the capture body, the ligand is preferably immobilized on the solid phase. The complex may be formed on the solid phase, by immobilizing the ligand on the solid phase. The ligand may have been immobilized on the solid phase in advance. Alternatively, the ligand may be immobilized on the solid phase during or after formation of the complex. When the ligand is immobilized on the solid phase in advance, the ligand and the solid phase may be covalently bonded directly or via a linker, for example. In yet another example of the detection method of the present embodiment, an example of detecting the TTR tetramer with use of the ligand immobilized on the solid phase, and the labeled anti-TTR antibody, will be described with reference to Figs.3A and B. The invention, however, is not limited thereto. Fig.3A represents a state before the complex is formed in the complex forming step. Fig.3B shows a state where a signal is generated by a labeling substance in the complex in the signal measurement step. In the example of Fig.3A, the ligand (20) is previously covalently bonded to the solid phase (40) via the linker (21). The labeling substance (50) is directly covalently bonded to the anti-TTR antibody (30) in advance. Referring to Fig.3B, the TTR tetramer (10) is captured on the solid phase, by binding of the ligand (20) immobilized on the solid phase (40) to the T4 binding site (11). Further, an anti-TTR antibody (30) that contains the labeling substance (50) binds to the TTR tetramer (10), so that a complex that contains the TTR tetramer (10), the ligand (20), the anti-TTR antibody (30), and the labeling substance (50) is formed on the solid phase (40). In Fig.3B, the labeling substance (50) is an enzyme. Signal (61) is generated by reacting substrate (60) with labeling substance (50), which is an enzyme. The TTR tetramer (10) in the sample may be detected by measuring the signal (61).

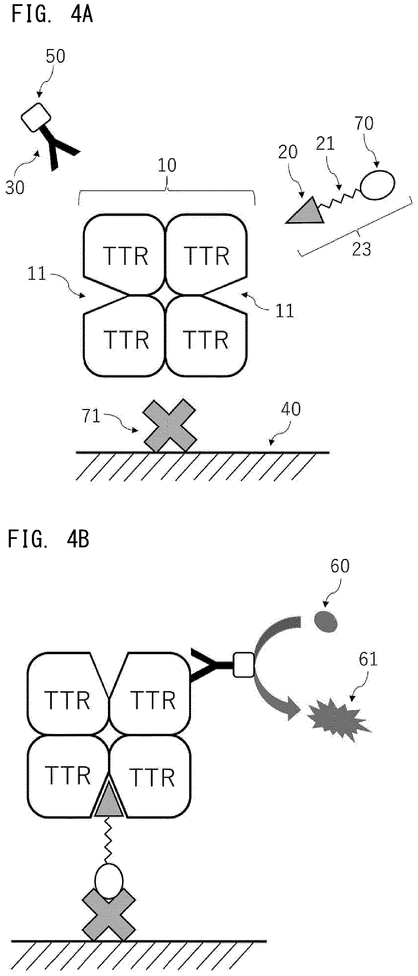

In yet another example of the detection method of the present embodiment, an example of detecting the TTR tetramer with use of the tagged probe, a solid phase that contains the binding partner, and a labeled anti-TTR antibody will be described with reference to Figs.4A and B. The invention, however, is not limited thereto. Fig.4 A represents a state before the complex is formed in the complex forming step. Fig.4 B represents a state in which a signal is generated by a labeling substance in the complex in the signal measurement step. In the example of Fig.4 A, the tagged probe (23) is used as the capture body. In the tagged probe (23), the ligand (20) and the tag (70) are covalently bonded to each other via a linker (21). On the solid phase (40), a binding partner (71) capable of specifically binding to the tag (70) is immobilized in advance by covalent bond. The labeling substance (50) is previously covalently bonded to the anti-TTR antibody (30). Referring to Fig.4 B, the ligand (20) in the tagged probe (23) binds to the T4 binding site (11). Further, the tagged probe (23) bound to the TTR tetramer (10) is immobilized on the solid phase (40) by binding of the tag (70) in the tagged probe (23) to the binding partner (71). Alternatively, the tagged probe (23) may be immobilized on the solid phase (40) by first binding the tag (70) to the binding partner (71). Thereafter, the tagged probe (23) on the solid phase and the TTR tetramer (10) may contact with each other, and the ligand (20) in the tagged probe (23) may bind to the T4 binding site (11). This captures the TTR tetramer (10) on the solid phase. Further, an anti-TTR antibody (30) that contains the labeling substance (50) binds to the TTR tetramer (10), so that a complex that contains the TTR tetramer (10), the ligand (20), the anti-TTR antibody (30), and the labeling substance (50) is formed on the solid phase (40). In Fig.3B, the labeling substance (50) is an enzyme. Signal (61) is generated by reacting substrate (60) with labeling substance (50), which is an enzyme. The TTR tetramer (10) in the sample may be detected by measuring the signal (61).

In this specification, "measuring the signal" includes quantitatively or semi-quantitatively outputting the signal intensity, not only quantifying the signal intensity, but also qualitatively or semi-quantitatively outputting the signal intensity." The term "determining" refers to determining that a measured value of the signal is "no signal" if the measured value of the signal is below a detection limit or below a predetermined threshold value, and "there is a signal" if the measured value is above a detection limit, and then outputting the result of the determination. The term "semi-quantitatively outputting the signal intensity" refers to determining which of a plurality of stages, such as "weak" and "strong", are included, based on the measured value of the signal.means outputting the result of determination.

The signal intensity reflects the amount of TTR tetramer captured by the capture body and bound by the detector. The measured value of the signal acquired by quantifying the signal intensity may therefore be a value indicating the amount of TTR tetramer detected from the sample. In the detection method of the present embodiment, the measured value of the signal is preferably acquired in the step of measuring the signal.

The method for measuring signal per se has been already known in the art. Appropriate measurement method may be selected depending on the type of signal attributable to the labeling substance. For an exemplary case where the signal is chemiluminescence or color generated by reaction of the enzyme with the substrate, the signal may be measured with a known instrument such as a spectrophotometer or a luminometer. In an exemplary case where the signal is radiation generated from a radioisotope, the signal may be measured with a known instrument such as a scintillation counter. When the signal is fluorescence generated from a fluorescent substance, the signal may be measured with a known instrument such as a fluorescence microplate reader. Excitation wavelength and fluorescence wavelength may properly be determined depending on the type of fluorescent substance employed.

In the detection methods of the present embodiment, B/F (Bound/Free) separation for removing any free component that remains unreacted without forming the complex may be performed between formation of the complex and measurement of the signal. The free component that remains unreacted refers to a component that does not constitute a complex. This is exemplified by anti-TTR antibody and ligand which remained unbound with the TTR tetramer. Technique for the B/F separation is not specially limited, and may be conducted, in an exemplary case where the solid phase is a particle, by centrifugation so as to collect only the solid phase having the complex bound thereon. With the solid phase given as a container such as a microplate or a microtube, the B/F separation may rely upon removal of a liquid that contains the unreacted free component. With the solid phase given as a magnetic particle, the B/F separation may rely upon removal of a liquid that contains the unreacted free component, under suction through a nozzle, while magnetically restraining the magnetic particle with use of a magnet. The method is preferred from the viewpoint of automatization. After removing the unreacted free components, the solid phase having the complex bound thereon may be washed with suitable aqueous medium such as PBS. The solid phase may be washed with a commercially available cleaning liquid such as HISCL (registered trademark) cleaning liquid.

Processes for preparing the compounds represented by formula (I) will be described below. The compound represented by formula (I) may be obtained, for example, by covalently bonding a compound represented by formula (VIII) below, a tag, a labeling substance, or a solid phase, via a linker. In Formula (VIII), R ¹, R ², R ¹, L ¹, and ring A are defined as in Formula (I) above. Q, R ⁴ and R ⁵ in ring A are also defined as in Formula (I) above.

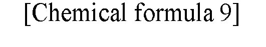

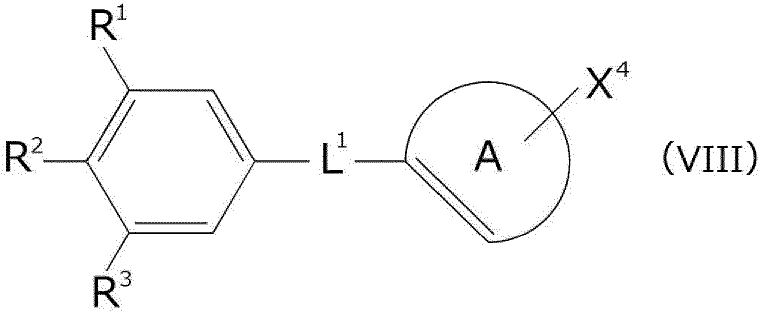

In the formula (VIII), X⁴ is -R⁶-NH₂, -R⁶-(C=O)-NH₂, -R⁶-NH-(C=O)-OH, -R⁶-(C=O)-H, -R⁶-(C=O)-OH, -R⁶-O-(C=O)-H, -R⁶-(C=S)-NH₂, -R⁶-NH-(C=S)-H, -R⁶-OH, -R⁶-SH, -NH-R⁷, -(C=O)-NH-R⁷, -NH-(C=O)-R⁷, -(C=O)-R⁷, -(C=O)-O-R⁷, -O-(C=O)-R⁷, - (C=S)-NH-R⁷, -NH-(C=S)-R⁷, -O-R⁷, -S-R⁷, hydrogen atom or halogen atom.

R ⁶ is defined as in Formula (I) above. R ⁷ each independently represents an alkyl group having 1 to 10 carbon atoms which may have a substituent, an aryl group having 6 to 12 carbon atoms which may have a substituent, a heteroaryl group having 4 to 12 carbon atoms which may have a substituent, a cycloalkyl group having 3 to 8 carbon atoms which may have a substituent, or a heterocycloalkyl group having 2 to 8 carbon atoms which may have a substituent,

When R ⁷ represents an alkyl group having 1 to 10 carbon atoms, such alkyl group is exemplified by methyl, ethyl, propyl, isopropyl, butyl, isobutyl, pentyl, neopentyl, hexyl, heptyl, octyl, 2-ethylhexyl, nonyl and decyl. Among them, an alkyl group having 1 to 4 carbon atoms is preferred. When R ⁷ represents an alkyl group having a substituent, the number of carbon atoms described above does not include the number of carbon atoms of the substituent.

When R ⁷ is an aryl group having 6 to 12 carbon atoms, such group is exemplified by phenyl, naphthyl and biphenylyl. When R ⁷ is a heteroaryl group having 4 to 12 carbon atoms, such group may only be an aromatic ring having 4 to 12 carbon atoms which contains one or more heteroatoms selected from N, S, O and P. This is exemplified by groups such as furanyl, pyrrole, thiophenyl, triazole, oxadiazole, pyridyl and pyrimidyl. When R ⁷ represents an aryl group or a heteroaryl group having a substituent, the number of carbon atoms described above does not include the number of carbon atoms of the substituent.

When R ⁷ is a cycloalkyl group having 3 to 8 carbon atoms, such group is exemplified by cyclopropyl, cyclobutyl, cyclopentyl, cyclohexyl, cycloheptyl and cyclooctyl. When R ⁷ is a heterocycloalkyl group having 2 to 8 carbon atoms, such group may only be a non-aromatic ring having 2 to 8 carbon atoms that contains one or more heteroatoms selected from N, S, O and P. This is exemplified by groups such as oxacyclopropyl, oxazolidinyl, pyrrolidinyl, piperidinyl and morpholinyl. When R ⁷ represents a cycloalkyl group or a heterocycloalkyl group having a substituent, the above carbon number does not include the carbon number of the substituent.

The substituent in the R ⁷ is exemplified by groups such as carboxy, cyano, alkoxy, nitro, =O,=S, -SH, halogen-atom, haloalkyl, heteroalkyl, carboxyalkyl, amine, amido and thioether. The R ⁷ may have a plurality of substituents. The alkyl group in alkoxy is a linear or branched saturated aliphatic hydrocarbon group having 1 to 5 carbon atoms, preferably 1 or 2 carbon atoms.

The compound represented by formula (VIII) is exemplified by thyroxine, thyroxine methyl, tafamidis, and stilbene derivative. Structural formulas of thyroxine, thyroxine methyl, and tafamidis are illustrated below.

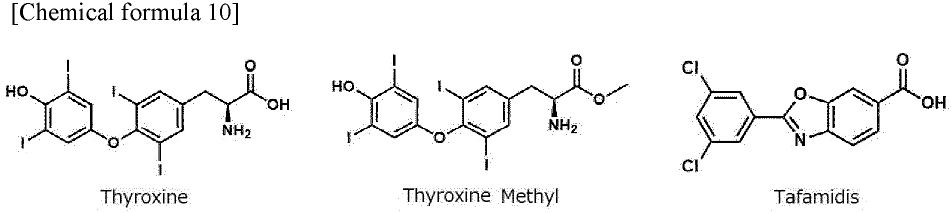

Preferred stilbene derivatives are stilbene derivatives 156 to 162,169 and 321 represented by the following structural formula. Among them, stilbene derivative 161 is particularly preferred.

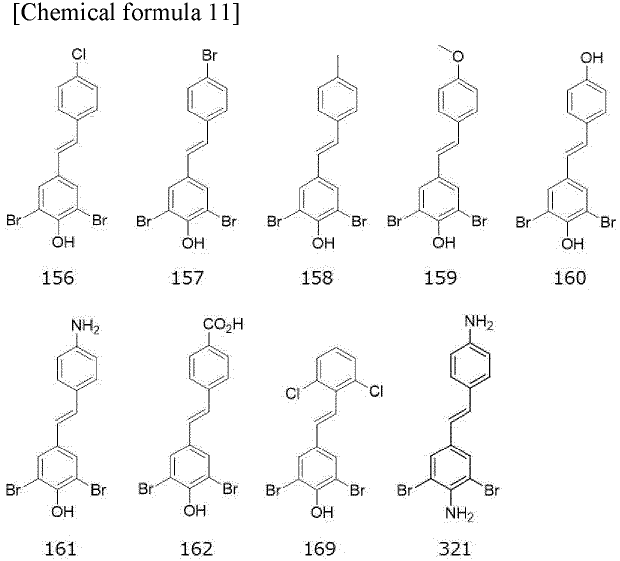

The labeling substance, the tag, or the solid phase is preferably covalently bonded to the X ⁴ in Formula (VIII) via a linker. The binding means is not specially limited, and for example, crosslinking utilizing a functional group is convenient and preferred. The functional group is preferably, but not limited to, amino group, carboxy group or sulfhydryl group, since a commercially available crosslinker may be used. The crosslinker is preferably a bifunctional linker having a reactive group and/or a functional group at both ends. The reactive group may properly be determined depending on the functional group possessed by the compound represented by formula (VIII), and the labeling substance, the functional group possessed by the tag or the solid phase. For an exemplary case where the compound represented by formula (VIII) has an amino group and the labeling substance has a sulfhydryl group, a bifunctional linker having an NHS ester and a maleimide group may be used. The labeling substance, the tag, or the solid phase, if it further contains a spacer arm having a reactive group at the end, may cause the reactive group to react with the functional group of the compound represented by formula (VIII). For adding biotin to the compound represented by formula (VIII), a commercially available biotin labeling reagent may be used. The reagent contains biotin having a spacer arm having a reactive group attached to the end. The spacer arm is, for example, a PEG chain. Alternatively, the labeling substance, the tag, or the solid phase, if it further contains a spacer arm having a reactive group at the end, may cause the reactive group to react with a functional group of the bifunctional linker. For an exemplary case where the compound represented by formula (VIII) has an amino group and the labeling substance further contains a spacer arm having a maleimide group at the end, a bifunctional linker having an NHS ester and a sulfhydryl group may be used.

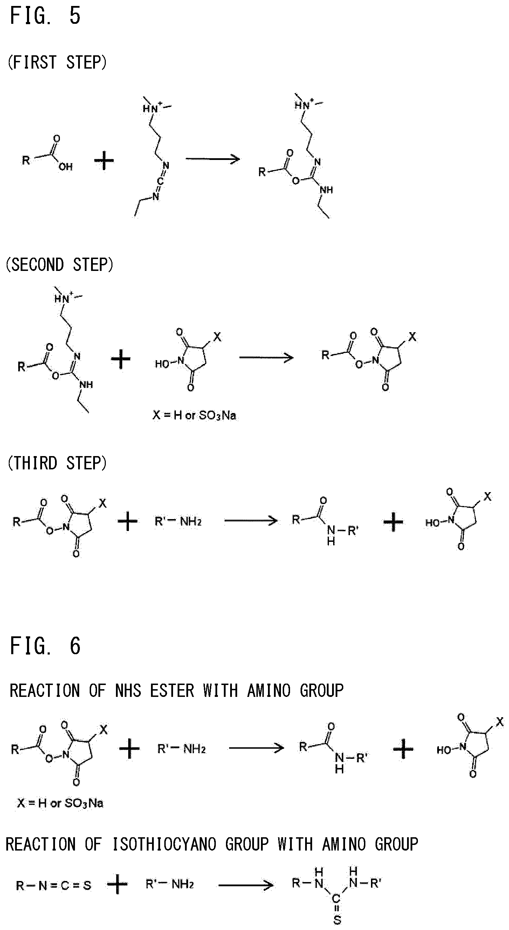

Reactions for crosslinking of typical functional groups will be described below. The substance having a carboxy group as the functional group may be bonded to the substance having an amino group as the functional group, through three steps illustrated in Fig.5. First, as illustrated in the first step in Fig.5, a substance having a carboxy group is reacted with a compound having a carbodiimide group (-N=C=N-) (in Fig.5, WSC). Next, as illustrated in the second step in Fig.5, the unstable NHS ester is formed by reacting the product of the first step with NHS. Then, as illustrated in the third step in Fig.5, the product of the second step may be crosslinked by reacting the product with a substance having an amino group. The crosslinking agent, when attached to the X ⁴ is a linker having an amino-group, a labeling substance, a tag, or a solid phase. The linker having an amino group at both ends may be used, when the compound represented by Formula (VIII) having a carboxy group on the X ⁴ is crosslinked with a labeling substance having a carboxy group, a tag or a solid phase.

The substance having an amino group as the functional group may cross-link with a substance having an NHS ester or an isothiocyano group as the reactive group, as illustrated in Fig.6. For an exemplary case where a compound represented by Formula (VIII) having an amino group in the X ⁴ is cross-linked with a labeling substance having an amino group, a tag, or a solid phase, then a linker having NHS-ester at both ends may be used.

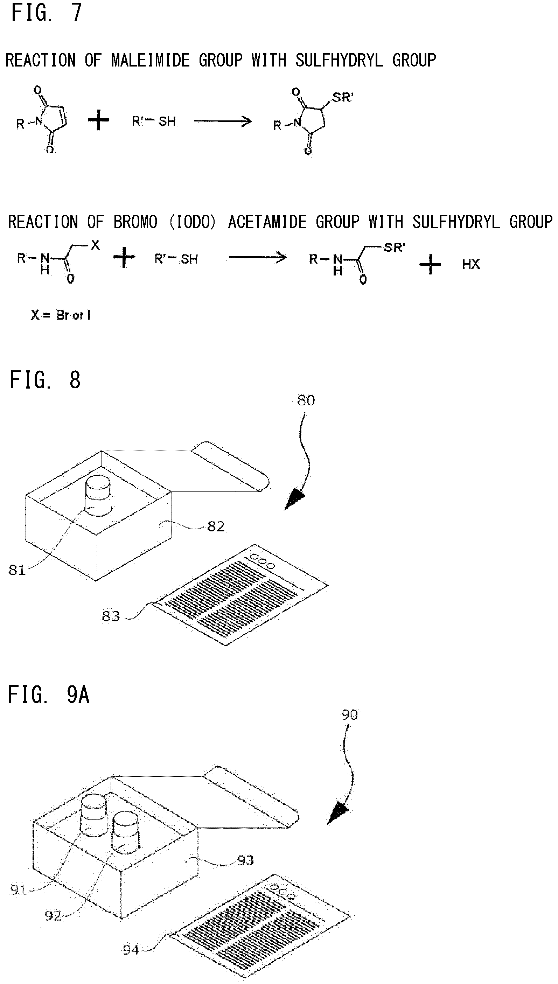

The substance having a sulfhydryl group as the functional group may cross-link with a substance having a maleimide group or a bromo (or iodo) acetamide group as the reactive group, as illustrated in Fig.7. For an exemplary case where a compound represented by Formula (VIII) having a sulfhydryl group on the X ⁴ is cross-linked with a labeling substance having a sulfhydryl group, a tag, or a solid phase, then a linker having maleimide at both ends may be used.

In an exemplary case where a substance having a carboxy group as the functional group and a substance having an amino group as the reactive group are covalently bonded, an amidation reaction with a known condensing agent may be utilized. Such condensing agent is exemplified by O-(7-azabenzotriazole-1-yl)-1,3-tetramethyluronium phosphonate (HATU), 4-(4,6-dimethoxy-1,5-triazine-2-yl)-4-methylmorpholinium chloride, 2-chloro-1,3-dimethylimidazolinium, 1H-benzotriazole-1-yloxytripyrrolidinone hexafluorophosphate, diphenylphosphoryl azide, and chlorotripyrrolidinone hexafluorophosphate, and N,N'-diisopropylcarbodiimide.

The crosslinking reaction and the amidation reaction may be conducted under ordinary temperature and pressure. The solvent used in the reaction is not particularly limited as long as it is inert to the reaction and can dissolve or disperse the respective substances used in the reaction. Examples of such solvent include aromatic hydrocarbons such as benzene, toluene, and xylene; ethers such as tetrahydrofuran (THF), diethyl ether, ethylene glycol dimethyl ether, and 1,4-dioxane; amides such as N,N-dimethylformamide (DMF); sulfoxides such as dimethylsulfoxide; and halogenated hydrocarbons such as dichloromethane and chloroform. These solvents may be used alone or as a mixture.

A further embodiment relates to a method for evaluating stability of TTR tetramer in a sample (also referred to as "evaluation method of this embodiment"). The evaluation method of this embodiment is a method for evaluating stability of TTR tetramer contained in a sample in vitro. In the evaluation method of the present embodiment, a first complex is formed that contains the TTR tetramer in the sample, the ligand, the anti-TTR antibody, and the labeling substance, and then a first signal generated by the labeling substance contained in the first complex is measured. Samples, ligands, anti-TTR antibody, and labeling substance are as described above. Details of formation of the first complex and measurement of the first signal are the same as those described for formation of the complex and measurement of the signal in the detection method of the present embodiment.

As will be described later in Example 1, even if the TTR monomer levels in the sample are the same, the measured value of the signal is different between the sample that contains the wild-type TTR, the sample that contains the mutant TTR (T119M), and the sample that contains the mutant TTR (V30M). Specifically, a measured value of the sample that contains the mutant TTR (T119M) was higher than that of the sample that contains the wild-type TTR. On the other hand, a measured value lower than that of the sample containing wild-type TTR (V30M) was obtained from the sample containing mutant TTR (V30M). Here, variant TTR (T119M) has been known to form a highly stable TTR tetramer, and variant TTR (V30M) has been known to form a less stable tetramer. As described previously, the unstable TTR tetramer cannot stably maintain its structure, and dissociates into a dimer and a monomer. That is, if the sample contains a mutant TTR that forms a tetramer with low stability, the amount of TTR tetramer in the sample (i.e., TTR tetramer level) will be lower than that of the sample that contains wild-type TTR. On the other hand, if the sample contains a mutant TTR that forms a highly stable tetramer, the TTR tetramer level of the sample will be increased than that of the sample that contains the wild-type TTR. The measured value of the first signal obtained by the evaluation method of the present embodiment may therefore serve as an index of the stability of the TTR tetramer in the sample.

In the evaluation method of the present embodiment, the total amount of TTR contained in the sample (total amount of monomer, dimer, and tetramer) is preferably further measured. The total amount of TTR contained in the sample is also referred to as "TTR level of the sample". Specifically, the second complex is first formed, comprising the TTR monomer, the TTR dimer, or the TTR tetramer in the sample, a capture antibody capable of binding to the TTR monomer, and a detection antibody that contains a labeling substance and is capable of binding to the TTR monomer. A second signal generated by the labeling substance in the second complex is then measured. The formation of the second complex and the measurement of the second signal correspond to sandwich ELISA in which the TTR monomer, the TTR dimer, and the TTR tetramer in the sample are used as the test substance. That is, the measured value of the second signal obtained by the evaluation method of the present embodiment is a value indicating the total amount of TTR contained in the sample. The measured value of the second signal is used for normalizing the measured value of the first signal, to obtain a corrected value regarding the amount of TTR tetramer.

The capture antibody is an anti-TTR antibody used as a capture body. The detection antibody is an anti-TTR antibody used as a detector. The detection antibody is preferably a labeled anti-TTR antibody. In an exemplary case where a monoclonal antibody is used as the capture antibody and the detection antibody, the capture antibody and the detection antibody preferably recognize epitopes different from each other, so as to avoid competition. Alternatively, at least one of the capture antibody and the detection antibody preferably uses a polyclonal antibody. The commercially available anti-TTR antibody may be used as the capture antibody and the detection antibody. The labeling substance contained in the detection antibody may be the same as or different from the labeling substance used for forming the first complex.

The reason why the corrected value regarding the amount of TTR tetramer is acquired will be described. First, the total amount of TTR contained in the biological sample may vary from subject to subject. For example, the total amount of TTR in the blood sample is reduced typically with inflammatory disease, undernutrition, or ATTR, and is increased typically with renal failure, and hyperthyroidism. The measured value of the first signal may vary depending on the TTR tetramer level of the sample. For example, a higher TTR tetramer level in the sample may increase the capture and detected TTR tetramer, resulting in a larger measured value of the first signal. Also, the lower the TTR tetramer level in the sample, the less TTR tetramer is captured and detected, and the smaller the measured value of the first signal may be. Here, the TTR tetramer level of the sample depends on the stability of the TTR tetramer in the sample, as described above. Further, the TTR tetramer level of the sample may vary depending on the total amount of TTR contained in the sample. For example, if the stability of the TTR tetramer is the same among a plurality of samples, the TTR tetramer level may relatively increase in a sample having a large total amount of TTR. This is because the monomer and dimer of TTR in the sample usually spontaneously form a tetramer. Therefore, in order to more accurately evaluate the stability of the TTR tetramer in the sample, the measured value of the first signal that represents the amount of TTR tetramer detected from the sample is preferably normalized to the measured value of the second signal that represents the total amount of TTR contained in the sample. Specifically, the corrected value regarding the amount of TTR tetramer is acquired by dividing the measured value of the first signal by the measured value of the second signal.

In a preferred embodiment, the stability of the TTR tetramer in the sample is evaluated based on a corrected value regarding the amount of the TTR tetramer. For example, the stability of the TTR tetramer in the sample may be evaluated based on a result of comparison between the corrected value regarding the amount of TTR tetramer and a predetermined threshold value. That is, the TTR tetramer in the sample may be evaluated to be stable if the corrected value is equal to or above a predetermined threshold value. The TTR tetramer in the sample may alternatively be evaluated to be unstable if the corrected value is below a predetermined threshold value.

The predetermined threshold value may be properly determined, without special limitation. For example, TTR tetramer in a biological sample obtained from each of a plurality of healthy subjects (healthy group) and a plurality of ATTR patients (patient group) is detected, and a measured value of the first signal is acquired. Also, a measured value of the second signal is acquired by measuring the total amount of TTR in the biological sample. Further, for each subject, a corrected value regarding the amount of TTR tetramer is acquired by dividing the measured value of the first signal by the measured value of the second signal. A value that can most accurately distinguish the healthy group and the patient group is then determined as the predetermined threshold value to be set. The threshold value is preferably determined, typically considering sensitivity, specificity, positive predictive value, and negative predictive value.

A further embodiment is a reagent (also referred to as "reagent of this embodiment") that contains a compound (ligand) capable of binding to the T4 binding site of the TTR tetramer. The reagent of the present embodiment is used in the detection method of the present embodiment and the evaluation method of the present embodiment. The reagent of this embodiment may contain a ligand (labeled probe) having a labeling substance covalently bonded thereto directly or via a linker. Alternatively, the reagent of the present embodiment may contain a ligand (tagged probe) having a tag covalently bonded thereto directly or via a linker. The reagent of the present embodiment preferably contains a compound represented by formula (I) described above. The ligand, tag, and labeling substance are as described above.

An exemplary reagent according to the present embodiment in the kit form is illustrated in Fig.8. In Fig.8, reference numeral 80 denotes a kit that contains the reagent of the present embodiment. Reference numeral 81 denotes a first vial filled with a reagent that contains a compound capable of binding to the T4 binding site of the TTR tetramer. Reference numeral 82 denotes a packaging box. 83 denotes a package insert. The package insert may typically describe methods of use, storage methods, and compositions of the reagents of the present embodiment.

A further embodiment is a reagent kit (also referred to as "reagent kit" in this embodiment) that contains a first reagent that contains a compound (ligand) capable of binding to the T4 binding site of the TTR tetramer, and a second reagent that contains an antibody capable of binding to the TTR monomer (anti-TTR antibody). The reagent kit of the present embodiment is used in the detection method of the present embodiment and the evaluation method of the present embodiment. In the reagent kit of the present embodiment, the first reagent may contain a ligand (labeled probe) having a labeling substance covalently bonded thereto directly or via a linker. Alternatively, the first reagent may contain a ligand (tagged probe) having a tag covalently bonded thereto directly or via a linker. In the reagent kit of the present embodiment, the first reagent preferably contains a compound represented by formula (I) described above. The ligand, anti-TTR antibody, tag, and labeling substance are as described above.

An exemplary reagent kit of this embodiment is illustrated in Fig.9A. In Fig.9 A, reference numeral 90 denotes a reagent kit of this embodiment. Reference numeral 91 denotes a first container filled with the first reagent. Reference numeral 92 denotes a second container filled with the second reagent. Reference numeral 93 denotes a packaging box. Reference numeral 94 denotes a package insert. The package insert may typically describe methods for using the reagent kit of the present embodiment, methods for storing the individual reagents, and compositions.

The reagent kit of the present embodiment, if the first reagent contains a tagged probe, may further contain a third reagent that contains a binding partner. For example, the reagent kit of the present embodiment contains a first reagent that contains a tagged probe, a second reagent that contains an anti-TTR antibody, and a third reagent that contains a binding partner covalently bonded to the labeling substance. Alternatively, the reagent kit of the present embodiment contains a third reagent that contains a first reagent that contains a tagged probe, a second reagent that contains a labeled anti-TTR antibody, and a binding partner covalently bound to the magnetic particle.

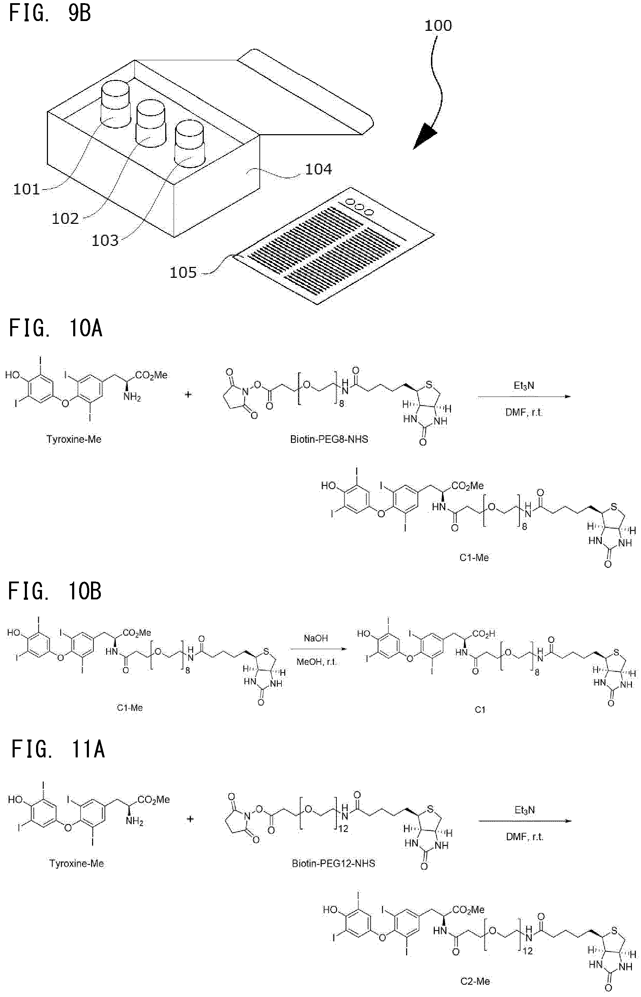

Another example of the reagent kit of this embodiment is illustrated in Fig.9B. In Fig.9 B, reference numeral 100 denotes a reagent kit of the present embodiment. Reference numeral 101 denotes a first container filled with the first reagent. Reference numeral 102 denotes a second container filled with the second reagent. Reference numeral 103 denotes a third container filled with the third reagent. Reference numeral 104 denotes a packaging box. 105 denotes a package insert. The package insert may typically describe methods for using the reagent kit of the present embodiment, methods for storing the individual reagents, and compositions.

The reagent kit of the present embodiment may further comprise a calibrator. The calibrator is exemplified by a buffer solution free of TTR (negative control), and a buffer solution containing a known concentration of recombinant TTR. The recombinant TTR may be a wild-type TTR, or a mutant TTR having known tetramer stability. Alternatively, the calibrator may be plasma or serum of a healthy person. The calibrator may also be a buffer solution that contains TTR derived from plasma of a healthy person or an ATTR patient.

The present invention will be further detailed below, referring to Examples, to which the present invention is by no means limited.

### EXAMPLES

### Preparation Example: Preparation of Tagged Probe

The compound capable of binding to the T4 binding site is exemplified by thyroxine methyl, tafamidis and stilbene derivatives. Six kinds of biotin-added probes (referred to as probes C1 to C6, respectively) were prepared by adding biotin as a tag to these compounds via a PEG linker. Probes C1 and C2 were biotin-PEGn-thyroxine (n=8 or 12), probes C3 and C4 were biotin-PEGn-Tafamidis (n=8 or 11), and probes C5 and C6 were biotin-PEGn-stilbene derivatives (n=8 or 12).

### (1) Preparation of probe C1 (biotin-PEG8-thyroxine)

In the first step, C1 methyl (C1-Me) was synthesized from thyroxine methyl. Fig.10 A shows the synthesis scheme in the first step. Referring to Fig.10 A, a 10 mL test tube was filled with biotin-PEG8-NHS (29.35 mg, 38.37 µmol) and thyroxine methyl (33.70 mg, 42.61 µ mol, 1.1 eq.), argon-substituted with Ar, and then dissolved in DMF (0.5 mL). To this solution was added a solution of triethylamine (Et ₃ N) (16 µL, 114.29 µ mol, 3 eq.) in DMF (0.5 mL), and stirred at room temperature for 18 hours. The reaction was followed by mixing the reaction liquid in CH ₂ Cl ₂, followed by thin-layer chromatography (TLC) (CH ₂ Cl ₂ / MeOH = 5:1, colored with phosphomolybdic acid), to confirm disappearance of the biotin-PEG8-NHS spot. After completion of the reaction, the solvent was distilled off to obtain a crude body. The crude product was purified by silica gel column chromatography (Wakogel C-400 HG, 10 g, CH Cl ₂ / MeOH = 1:0 to 10:1), to obtain C1 methyl (C1-Me) (39.76 mg, 72.0%). Measured results of NMR of C1-Me are shown below.

¹H NMR (400 MHz, DMSO-d₆) δ9.28 (s, 1H), 8.37 (d, J = 8.1 Hz, 1H), 7.82 (t, J = 5.5 Hz, 1H), 7.80 (s, 2H), 7.05 (s, 2H), 6.41 (brs, 1H), 6.35 (brs, 1H), 4.55-4.49 (m, 1H), 4.32-4.28 (m, 1H), 4.14-4.10 (m, 1H), 3.62, (s, 3H), 3.53-3.47 (m, 30H), 3.39 (t, J = 5.9 Hz, 2H), 3.18 (q, J = 5.8 Hz, 2H), 3.11-3.01 (m, 2H), 2.86-2.79 (m, 2H), 2.59 (brs, 1H), 2.33 (t, J = 6.8 Hz, 2H), 2.06 (t, J = 7.4 Hz, 2H), 1.64-1.56 (m, 1H), 1.53-1.41 (m, 3H), 1.34-1.23 (m, 2H).

¹³C NMR (101 MHz, DMSO-d₆) δ172.1, 171.7, 170.0, 162.7, 160.0, 151.4, 151.0, 140.7, 139.1, 125.0, 91.6, 87.7, 69.8, 69.7, 69.64, 69.56, 69.2, 61.0, 59.2, 55.4, 54.9, 52.9, 51.9, 38.4, 35.9, 35.1, 34.9, 28.2, 28.0. 25.25, 25.22.

In the second step, probe C1 was synthesized from C1-Me. Fig.10 B shows a synthesis scheme in the second step. Referring to Fig.10 B, C1-Me (36.65 mg, 25.44 µmol) and MeOH (1 mL) were placed in a 10 mL test tube, and the tube was dissolved at room temperature. To this solution was added 0.05 M NaOH (1.1 mL, 55 µ mol, 2.2 eq.), and stirred at room temperature for 1 hour. The reaction was followed by adding the reaction liquid to 1 M HCl, adding MeOH until the resultant white turbidity dissolved, and then confirming disappearance of the C1-Me spot with reverse phase TLC (MeCN/H ₂ O = 1:1, UV254 nm). After completion of the reaction, MeOH was distilled off. To the obtained residue was added 0.05 M HCl (1.4 mL, 70 µmol, until the pH reached 3 or below), to obtain a white turbidity. After stirring for 14 hours at room temperature, the mixture was distilled off to obtain a crude product (40.05 mg). The crude product was purified by reverse phase silica gel chromatography (Cosmosil 75C18-OPN, 10 g, MeCN/H O = 1:1), to obtain probe C1 (22.4 mg, 61.7%). Measured results of NMR of probe C1 are shown below.

¹H NMR (400 MHz, DMSO-d₆) δ12.83 (brs, 1H), 9.28 (brs, 1H), 8.20 (d, J = 8.3 Hz, 1H), 7.82 (t, J = 5.6 Hz, 1H), 7.79 (s, 2H), 7.05 (s, 2H), 6.41 (brs, 1H), 6.35 (brs, 1H), 4.48-4.42 (m, 1H), 4.32-4.28 (m, 1H), 4.14-4.10 (m, 1H), 3.53-3.47 (m, 30H), 3.39 (t, J = 5.9 Hz, 2H), 3.18 (q, J = 5.8 Hz, 2H), 3.11-3.04 (m, 2H), 2.84-2.76 (m, 2H), 2.57 (d, J = 12.4 Hz, 1H), 2.36-2.31 (m, 2H), 2.08-2.04 (m, 2H), 1.64-1.56 (m, 1H), 1.53-1.43 (m, 3H), 1.34-1.23 (m, 2H).

¹³C NMR (101 MHz, DMSO-d₆) δ172.5, 172.1, 169.8, 162.7, 151.3, 151.0, 140.7, 139.6, 125.0, 91.5, 87.7, 69.8, 69.7, 69.63, 69.57, 69.2, 66.9, 61.0, 59.2, 55.4, 52.8, 38.4, 36.0, 35.1, 28.2, 28.0, 25.3.

### (2) Preparation of probe C2 (biotin-PEG12-thyroxine)

In the first step, C2 methyl (C2-Me) was synthesized from thyroxine methyl. Fig.11A illustrates a synthesis scheme in a first step. Referring to Fig.11 A, a 10 mL test tube was filled with biotin-PEG12-NHS (58.58 mg, 62.25 µmol), and thyroxine methyl (58.52 mg, 74.00 µ mol, 1.1 eq.), followed by Ar-displacement, and then DMF (1 mL) was added thereto to dissolve. To this solution was added a solution of Et ₃ N (26 µL, 186.54 µ mol, 3 eq.) in DMF (1 mL), and stirred at room temperature for 22 hours. The reaction was followed by mixing the reaction liquid with a CH ₂ Cl ₂, followed by confirmation of disappearance of the biotin-PEG12-NHS spot with TLC (CH ₂ Cl ₂ to MeOH = 5:1, colored with phosphomolybdic acid). After completion of the reaction, the solvent was distilled off to obtain a crude body. The crude product was purified by silica gel column chromatography (Wakogel C-400 HG, 10 g, CH Cl ₂ / MeOH = 1:0 to 10:1), to obtain C2methyl (C2-Me) (39.62 mg, 39.4%). Measured results of NMR of C2-Me are shown below.

¹H NMR (400 MHz, DMSO-d₆) δ9.28 (s, 1H), 8.37 (d, J = 8.0 Hz, 1H), 7.83 (t, J = 5.2 Hz, 1H), 7.80 (s, 2H), 7.05 (s, 2H), 6.41 (brs, 1H), 6.34 (brs, 1H), 4.55-4.49 (m, 1H), 4.32-4.28 (m, 1H), 4.14-4.10 (m, 1H), 3.62, (s, 3H), 3.50-3.47 (m, 46H), 3.39 (t, J = 5.9 Hz, 2H), 3.18 (q, J = 5.8 Hz, 2H), 3.11-3.01 (m, 2H), 2.86-2.79 (m, 2H), 2.57 (d, J = 12.4 Hz, 1H), 2.33 (t, J = 6.8 Hz, 2H), 2.06 (t, J = 7.4 Hz, 2H), 1.64-1.56 (m, 1H), 1.54-1.41 (m, 3H), 1.35-1.23 (m, 2H).

¹³C NMR (101 MHz, DMSO-d₆) δ172.1, 170.0, 162.7, 150.7, 150.2, 140.7, 139.1, 137.0, 136.8, 125.1, 95.7, 92.3, 91.6, 87.7, 69.8, 69.64, 69.56, 69.2, 61.0, 59.2, 55.4, 54.9, 52.9, 52.8, 51.9, 38.4, 35.9, 35.1, 34.0, 28.2, 28.0. 25.3.

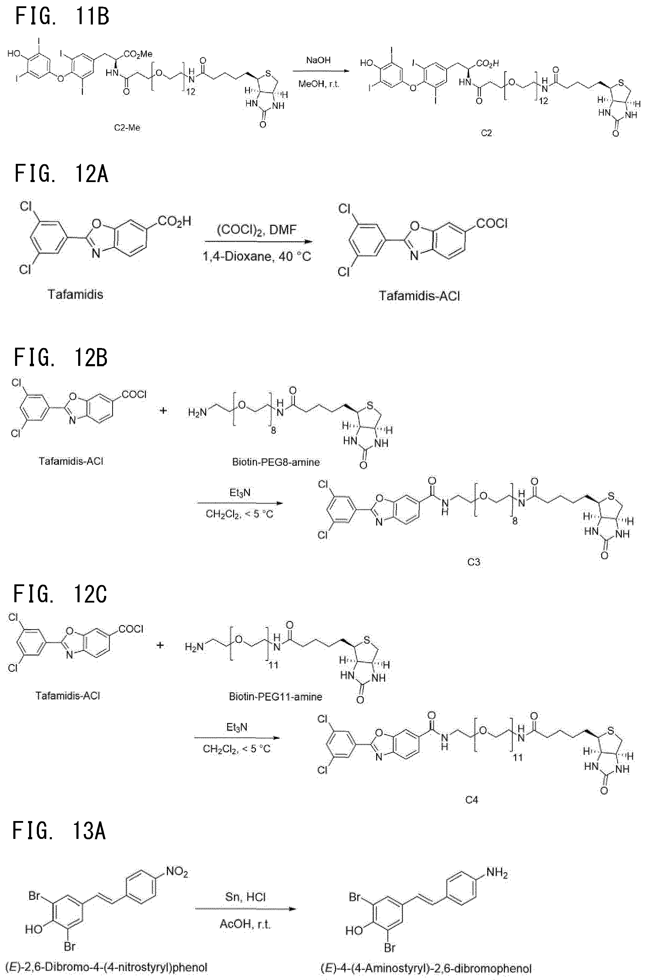

In a second step, probe C2 was synthesized from C2-Me. Fig.11B shows a synthesis scheme in the second step. Referring to Fig.11 B, C2-Me (34.83 mg, 21.54 µmol) and MeOH (1 mL) were placed in a 10 mL test tube, and the tube was dissolved at room temperature. To this solution was added 0.05 M NaOH (1 mL, 50 µ mol, 2.2 eq.), and stirred at room temperature for 1 hour. The reaction was followed by adding the reaction liquid to 1 M HCl, adding MeOH until the resultant white turbidity dissolved, and then confirming disappearance of the C2-Me spot with reverse phase TLC (MeCN/H ₂ O = 1:1, UV254 nm). After completion of the reaction, MeOH was distilled off. To the obtained residue was added 0.05 M HCl (1.6 mL, 80 µmol, until the pH became equal to or below 3), to obtain a white turbidity. After stirring for 17 hours at room temperature, the mixture was distilled off to obtain a crude product (34.47 mg). The crude product was purified by reverse phase silica gel chromatography (Cosmosil 75C - OPN, 10 g, MeCN/H to ₂ O = 1:1), to obtain probe C2 (23.95 mg, 69.4%). Measured results of NMR of probe C2 are shown below.

¹H NMR (400 MHz, DMSO-d₆) δ12.83 (brs, 1H), 9.28 (brs, 1H), 8.21 (d, J = 8.2 Hz, 1H), 7.82 (t, J = 5.7 Hz, 1H), 7.79 (s, 2H), 7.05 (s, 2H), 6.41 (brs, 1H), 6.35 (brs, 1H), 4.48-4.43 (m, 1H), 4.32-4.28 (m, 1H), 4.14-4.10 (m, 1H), 3.50-3.47 (m, 46H), 3.39 (t, J = 5.9 Hz, 2H), 3.18 (q, J = 5.8 Hz, 2H), 3.11-3.04 (m, 2H), 2.84-2.76 (m, 2H), 2.57 (d, J = 12.4 Hz, 1H), 2.36-2.31 (m, 2H), 2.06 (t, J = 7.4 Hz, 2H), 1.65-1.55 (m, 1H), 1.54-1.41 (m, 3H), 1.35-1.24 (m, 2H).

¹³C NMR (101 MHz, DMSO-d₆) δ172.5, 172.1, 169.8, 162.7, 151.3, 150.2, 140.7, 125.0, 91.5, 87.7, 69.8, 69.7, 69.63, 69.57, 69.2, 66.9, 61.0, 59.2, 55.4, 52.8, 38.4, 36.0, 35.1, 28.2, 28.0, 25.3.

### (3) Preparation of Probe C3 (biotin-PEG8-Tafamidis)

In the first step, Tafamidis-ACl was synthesized from Tafamidis. Fig.12A shows a synthesis scheme in the first step. Referring to Fig.12A, a 6-mL vial was charged with Tafamidis (98 mg, 0.32 mmol), 1.4-dioxane (1 mL), and DMF (1 µL, 0.01 mmol, 3 mol %), and stirred at room temperature (suspended). To this suspension was added oxalyl chloride (48 µL, 0.56 mmol, 1.75 eq), and stirred at 40° C. for 6 hours. Reaction follow-up was as follows. The reaction liquid was dissolved in MeOH/Et3N (100:1), and the solvent was then evaporated. The obtained residue was dissolved in DMSO-d6, and examined by ¹ H-NMR for disappearance of the tafamidis peak, and confirmation of a methyl ester of tafamidis-ACl (tafamidis-Me). After completion of the reaction, the mixture was concentrated in vacuo to obtain Tafamidis-ACl (92 mg, 88%). The ¹ H NMRs of Tafumizus-Me and Tafumizus-ACl were measured below.

### Tafamidis-Me

¹H NMR (400 MHz, DMSO-d₆) δ8.30 (d, J = 1.4 Hz, 1H), 8.14 (d, J = 1.9 Hz, 2H), 8.04 (dd, J = 8.4, 1.4 Hz, 1H), 7.97-7.94 (m, 2H), 3.91 (s, 3H).

### Tafamidis-ACl

¹H NMR (400 MHz, DMSO-d₆) δ8.27 (d, J = 1.4 Hz, 1H), 8.15 (d, J = 1.9 Hz, 2H), 8.03 (dd, J = 8.4, 1.4 Hz, 1H), 7.94 (t, J = 1.9 Hz, 1H), 7.92 (d, J = 8.4 Hz, 1H).

In a second step, probe C3 was synthesized from Tafamidis-ACl. Fig.12B shows a synthesis scheme in the second step. Referring to Fig.12B, a 10 mL test tube was charged with biotin-PEG8-amine (29.1 mg, 46 µmol), and CH-Cl ₂ (1 mL), and dissolved at room ₂. Et ₃ N (12.7 µL, 91 µ mol, 2 eq.) was added to the solution, and the mixture was ice-cooled to adjust the temperature to 5° C. or below. A suspension of Tafamidis-ACl (17.9 mg, 55 µ mol, 1.2 eq.) in CH ₂ Cl ₂ (1.5 mL) previously synthesized under ice-cooling was slowly added dropwise at 5° C. or below. The mixture was stirred at 5° C. or below for 1 hour, and then placed in a separatory funnel filled with H ₂ O (20 mL). After extracting with ethyl acetate (AcOEt), the organic layer was washed with Brine, and dried over Na ₂ SO ₄. The organic layer was evaporated in a solvent to obtain a crude body (48.4 mg). The crude product was purified by silica gel column chromatography (Silica Gel 60N 40-50 µ m, 10 g, CH Cl ₂ / MeOH = 8:1 to 1:1), to obtain probe C3 (34.8 mg, 81.4%). Measured results of NMR of probe C3 are shown below.

¹H NMR (400 MHz, CDCl₃) δ8.18 (d, J = 1.1 Hz, 1H), 8.16 (d, J = 1.9 Hz, 2H), 7.89 (dd, J = 8.3, 1.4 Hz, 1H), 7.80 (d, J = 8.3 Hz, 1H), 7.55 (t, J = 1.9 Hz, 1H), 7.36 (brs, 1H), 6.55 (brs, 1H), 5.74 (brs, 1H), 5.03 (brs, 1H), 4.52-4.48 (m, 1H), 4.33-4.30 (m, 1H), 3.72-3.60 (m, 32H), 3.56 (t, J = 5.0 Hz, 2H), 3.45-3.42 (m, 2H), 3.14 (td, J = 7.3, 4.7 Hz, 1H), 2.91 (dd, J = 12.8, 5.0 Hz, 1H), 2.73 (d, J = 12.8 Hz, 1H), 2.21 (td, J = 7.2, 2.6 Hz, 2H), 1.77-1.60 (m, 4H), 1.48-1.40 (m, 2H).

¹³C NMR (101 MHz, CDCl₃) δ173.1, 166.7, 163.3, 162.3, 150.8, 144.2, 136.0, 132.8, 131.8, 129.5, 126.1, 124.2, 120.1, 110.6, 77.3, 70.58, 70.56, 70.51, 70.48, 70.3, 70.2, 69.92, 69.86, 61.8, 60.1, 55.4, 40.6, 40.2, 39.2, 35.9, 28.1, 25.5.

### (4) Preparation of Probe C4 (Biotin-PEG11-Tafamidis)

Tafamidis-ACl was synthesized in the same manner as in the first step of production of probe C3. Probe C4 was synthesized from Tafamidis-ACl, according to the synthesis scheme illustrated in Fig.12C. Referring to Fig.12C, a 10 mL test tube was filled with biotin-PEG11-amine (40 mg, 52 µmol), and CH-Cl ₂ (1 mL) to dissolve at room ₂. Et- ₃ N (14.5 µL, 104 µ mol, 2.0 eq.) was added to the solution, and the mixture was ice-cooled to adjust the temperature to 5° C. or below. A suspension of Tafamidis-ACl (20.4 mg, 62 µ mol, 1.2 eq.) in CH ₂ Cl ₂ (2.0 mL) previously synthesized under ice-cooling was slowly added dropwise at 5° C. or below. The mixture was stirred at 5° C. or below for 1 hour, and then placed in a separatory funnel filled with H ₂ O (20 mL). After extracting with AcOEt, the organic layer was washed with Brine, and dried over Na ₂ SO ₄. The organic layer was evaporated in a solvent to obtain a crude body (44.8 mg). The crude product was purified by silica gel column chromatography (Wakogel C-400 HG, 8 g, CH Cl ₂ / MeOH = 10:1 to 6:1), to obtain probe C4 (26.5 mg, 48.0%). Measured results of NMR of probe C4 are shown below.

¹H NMR (400 MHz, CDCl₃) δ8.16 (d, J = 1.0 Hz, 1H), 8.13 (d, J = 1.9 Hz, 2H), 7.88 (dd, J = 8.3, 1.5 Hz, 1H), 7.77 (d, J = 8.4 Hz, 1H), 7.52 (t, J = 2.0 Hz, 1H), 7.36 (brs, 1H), 6.80 (t, J = 5.5 Hz, 1H), 6.40 (brs, 1H), 5.56 (brs, 1H), 4.49-4.46 (m, 1H), 4.30-4.27 (m, 1H), 3.72-3.58 (m, 44H), 3.54 (t, J = 5.1 Hz, 2H), 3.45-3.37 (m, 2H), 3.10 (td, J = 7.4, 4.6 Hz, 1H), 2.87 (dd, J = 12.9, 5.2 Hz, 1H), 2.72 (d, J = 12.7 Hz, 1H), 2.22 (t, J = 7.1 Hz, 2H), 1.78-1.59 (m, 4H), 1.45-1.38 (m, 2H).

¹³C NMR (101 MHz, CDCl₃) δ173.3, 166.6, 163.9, 162.2, 150.7, 144.1, 135.9, 132.8, 131.7, 129.5, 126.3, 126.1, 124.2, 120.0, 110.6, 77.3, 70.5, 70.44, 70.39, 70.2, 70.1, 70.0, 69.8, 61.8, 60.2, 55.6, 40.5, 40.1, 39.2, 35.9, 29.7, 28.2, 28.1, 25.6.

### (5) Production of Probe C5 (Biotin-PEG8-stilbene Derivative)

In the first step, (E)-4-(4-aminostyryl)-2,6-dibromo-4-(4-nitrostyryl)phenol was synthesized from (E)-2,6-dibromo-4-(4-nitrostyryl)phenol. Fig. 13 A shows the synthesis scheme in the first step. Referring to Fig. 13 A, in a 30 mL flask, (E)-2,6-dibromo-4-(4-nitrostyryl)phenol (680 mg, 1.70 mmol), acetic acid (AcOH) (6.8 mL), and hydrochloric acid (0.68 mL, 7.69 mmol, 4.5 eq.) were added, followed by suspension. Sn-powder (809 mg, 6.82 mmol, 4.0 eq.) was added, and the mixture was stirred at room temperature for 2 hours. The reaction liquid was placed in a separation funnel filled with H ₂ O (30 mL). After extracting twice with AcOEt (50 mL), the organic layer was washed three times with saturated NaHCO ₃ solutions (30 mL). The organic layers were washed with Brine, and dried over Na- ₂ SO- ₄. The organic layer was evaporated in a solvent to obtain a crude body (650 mg). The crude product was purified by silica gel column chromatography (Silica Gel 60 N, 60 g, hexane/AcOEt=4:1 to 1:1), to obtain yellow crystal (564 mg). Recrystallization with hexane/AcOEt (2:1, 33 mL) gave (E)-4-(4-aminostyryl)-2,6-dibromophenol (406 mg, 64.7%). (E) NMR measurement results of 4-(4-aminostyryl)-2,6-dibromophenol are shown below.

¹H NMR (400 MHz, CDCl₃) δ9.83 (s, 1H), 7.68 (s, 2H), 7.23 (d, J = 8.4 Hz, 2H), 7.01 (d, J = 16.4 Hz, 1H), 6.75 (d, J = 16.4 Hz, 1H), 6.54 (d, J = 8.5 Hz, 2H), 5.32 (brs, 2H).

¹³C NMR (101 MHz, CDCl₃) δ148.93, 148.86, 133.2, 129.4, 129.1, 127.6, 124.5, 119.7, 113.8, 112.5.

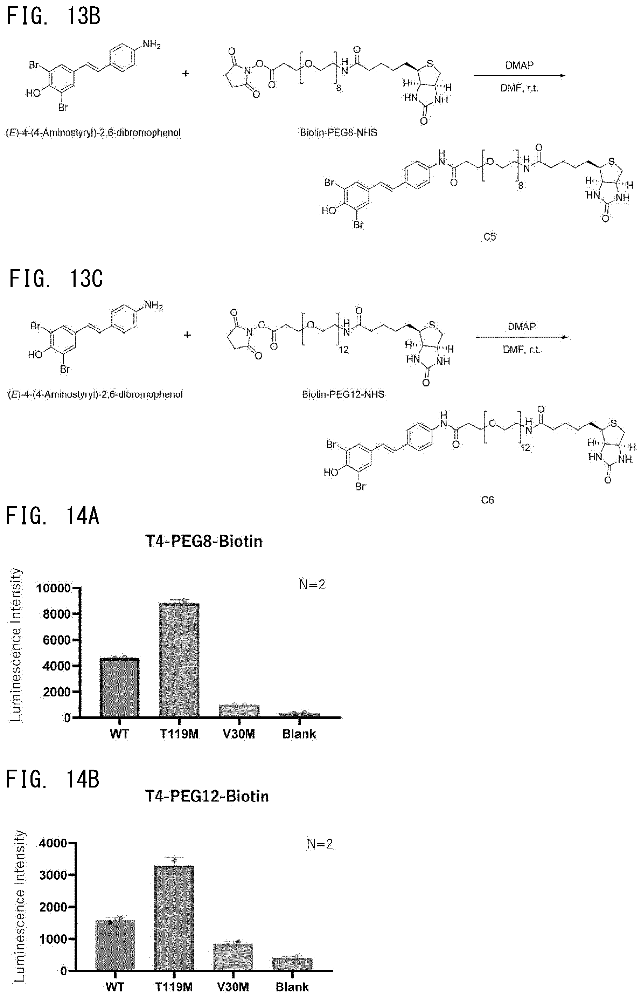

In a second step, probe C5 was synthesized from (E)-4-(4-aminostyryl)-2,6-dibromophenol. Fig. 13B shows a synthesis scheme in the second step. Referring to Fig. 13 B, biotin-PEG8-NHS (125 mg, 163 µmol), and (E)-4-(4-aminostyryl)-2,6-dibromophenol (120 mg, 326 µ mol, 2.0 eq.) were placed in a 10 mL test tube, and DMF (3.1 mL) was dissolved at room temperature. To this solution was added 4-dimethylaminopyridine (DMAP) (51.8 mg, 424 µ mol, 2.6 eq.), and stirred at room temperature for 43 hours. Evaporation of the solvent gave the crude body (246 mg). The crude product was purified by silica gel column chromatography (Wakogel C-400 HG, 24 g, CH Cl ₂ / MeOH = 10:1 to 3:1), to obtain probe C5 (26.6 mg, 16.0%). Measured results of NMR of probe C5 are shown below.

¹H NMR (400 MHz, CDCl₃) δ9.07 (s, 1H), 7.60 (d, J = 8.5 Hz, 2H) 7.56 (s, 2H), 7.38 (d, J = 8.6 Hz, 2H), 6.89 (d, J = 16.2, 1H), 6.81-6.77 (m, 2H), 6.38 (brs, 1H), 5.65 (brs, 1H), 4.49-4.46 (m, 1H), 4.30-4.26 (m, 1H), 3.82 (t, J = 5.6 Hz, 2H), 3.69-3.59 (m, 29H), 3.54 (t, J = 5.1 Hz, 2H), 3.43-3.37 (m, 2H), 3.10 (td, J = 7.3, 4.6 Hz, 1H), 2.88 (dd, J = 12.8, 5.0 Hz, 1H), 2.72 (d, J = 12.8 Hz, 1H), 2.67 (t, J = 5.7 Hz, 2H), 2.22 (t, J = 7.3 Hz, 2H), 1.77-1.57 (m, 4H), 1.46-1.36 (m, 2H).

¹³C NMR (101 MHz, CDCl₃) δ173.4, 170.2, 163.7, 148.9, 138.4, 132.7, 132.2, 129.7, 128.7, 127.0, 124.2, 120.1, 110.6, 77.2, 70.50, 70.47, 70.43, 70.41, 70.33, 70.30, 70.1, 70.0, 67.2, 61.8, 60.2, 55.5, 40.5, 39.2, 38.0, 35.9, 34.9, 28.2, 28.1, 25.6.

### (6) Preparation of probe C6 (biotin-PEG12-stilbene derivative)

(E)-4-(4-aminostyryl)-2,6-dibromophenol was synthesized in the same manner as in the first step of production of probe C5. Probe C6 was synthesized from (E)-4-(4-aminostyryl)-2,6-dibromophenol, according to the synthesis scheme illustrated in Fig.13C. Referring to Fig. 13 C, a biotin-PEG12-NHS (96 mg, 102 µmol), and (E)-4-(4-aminostyryl)-2,6-dibromophenol (75.2 mg, 204 µ mol, 2.0 eq.) were placed in a 10 mL test tube, and DMF (2.5 mL) was dissolved at room temperature. DMAP. (32.3 mg, 264 µ mol, 2.6 eq.) was added to the solution, and the mixture was stirred at room temperature for 24 hours. Evaporation of the solvent gave the crude body (210 mg). The crude product was purified by silica gel column chromatography (Wakogel C-400 HG, 24 g, CH Cl ₂ / MeOH = 10:1 to 3:1), to obtain probe C6 (48.0 mg, 39.4%). Measured results of NMR of probe C6 are shown below.

¹H NMR (400 MHz, CDCl₃) δ8.97 (s, 1H), 7.59 (d, J = 8.5 Hz, 2H), 7.56 (s, 2H), 7.39 (d, J = 9.0 Hz, 2H), 6.90 (d, J = 16.2 Hz, 1H), 6.80 (d, J = 16.2 Hz, 1H), 6.76 (t, J = 5.5 Hz, 1H), 6.26 (brs, 1H), 5.59 (brs, 1H), 4.50-4.47 (m, 1H), 4.31-4.28 (m, 1H), 3.82 (t, J = 5.5 Hz, 2H), 3.69-3.59 (m, 45H), 3.54 (t, J = 5.0 Hz, 2H), 3.43-3.40 (m, 2H), 3.13 (td, J = 7.3, 4.6 Hz, 1H), 2.88 (dd, J = 12.8, 4.9 Hz, 1H), 2.73 (d, J = 12.8 Hz, 1H), 2.66 (t, J = 5.7 Hz, 2H), 2.22 (t, J = 7.3 Hz, 2H), 1.78-1.59 (m, 4H), 1.46-1.38 (m, 2H).

¹³C NMR (101 MHz, CDCl₃) δ173.4, 172.7, 170.2, 164.0, 148.8, 138.3, 132.7, 132.2, 129.7, 128.7, 127.0, 124.1, 120.1, 110.5, 77.3, 70.46, 70.40, 70.37, 70.27, 70.25, 70.0, 69.9, 67.1, 66.5, 61.8, 60.2, 55.5, 40.5, 39.2, 37.9, 35.8, 34.8, 28.1, 28.0, 25.5, 25.4.

### EXAMPLE1: Detection of TTR tetramer in a sample (1)

The TTR tetramer in the sample containing the recombinant TTR and the blood sample was detected with use of the tagged probe, the labeling substance that contains the binding partner, and the solid phase having the anti-TTR antibody immobilized thereon.

### (1) sample

### (1.1) Samples Containing Recombinant TTR

Transthyretin (wild-type) human, recombinant (AlexoTech, T-500-10), transthyretin (T119M) human, recombinant (AlexoTech, T-515-10), and transthyretin (V30M) human, and recombinant (AlexoTech, T-505-10) were each diluted with 1% BSA/0.5% casein/PBS. This prepared a sample that contains recombinant TTR of wild-type (WT), stabilized variant (T119M), and destabilized variant (V30M) at a concentration of 100 ng/mL. Each recombinant TTR naturally formed a homotetramer in the sample.

### (1.2) Blood sample

Six serum samples were prepared by serially diluting commercially available serum (ProMedex Corporation, 12011378) with 1% BSA/0.5% casein/PBS. Also six plasma samples were prepared by serially diluting a commercially available plasma mix with 1% BSA/0.5% casein/PBS.

### (2) reagent

A polyclonal rabbit anti-human prealbumin antibody (Agilent Inc., A0002) was used as the antibody capable of binding to the TTR monomer. The antibody was diluted with PBS to prepare an anti-TTR antibody solution (2 µg/mL). Probes C1, C2, C5 and C6 were diluted with 1% BSA/0.5% casein/PBS, respectively, to prepare a probe solution (10 µM). The labeling substance was streptavidin-HRP (R&D Systems). The streptavidin-HRP was diluted 200 times with 1% BSA/0.5% casein/PBS, to prepare an HRP solution. ELISA-Star (trademark) peroxidase chemiluminescent substrate (Fujifilm Wako Pure Chemical Company, Ltd., 293-78804) was used as the substrate solution of HRP. A black 96-well plate (Sumitomo Bakelite Corporation) was used as the solid phase.

### (3) Detection of TTR tetramer

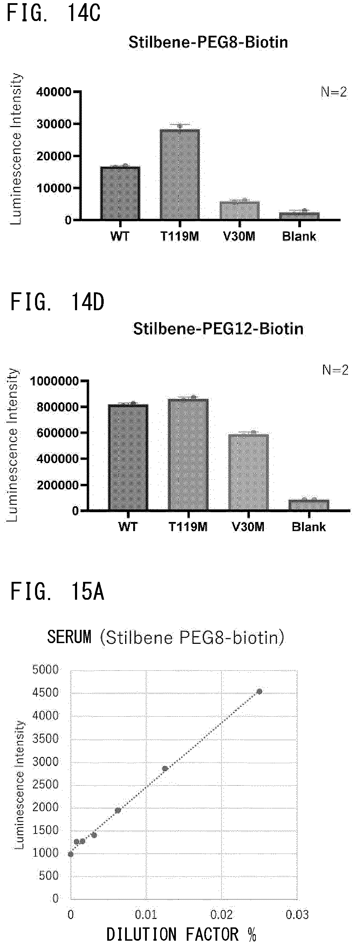

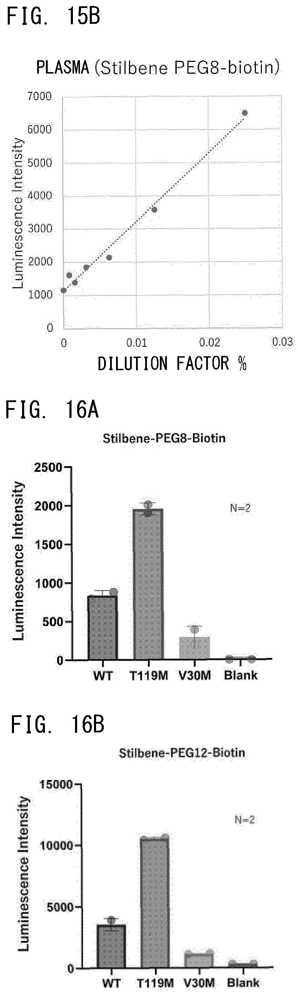

To each well of the plate, 100 µL of the anti-TTR antibody solution was added, followed by incubation at 4° C. overnight. The solution in the wells was discarded, and each well was washed with HISCL (registered trademark) washing solution (Sysmex Corporation). To each well, 300 µL of a blocking solution (1% BSA/0.5% casein/PBS) was added, followed by incubation for 1 hour at room temperature. The solution in the wells was discarded, and 100 µL of each sample was added to each well. The plate was incubated with stirring with a shaker at room temperature for 1 hour. As a control (blank), 100 µL of 1% BSA/0.5% casein/PBS was added to the wells, instead of the sample, and the wells were incubated in the same manner. The solution in the wells was discarded, and each well was washed with HISCL washing solution. 100 µL of the probe solution was added to each well, and the wells were incubated for 1 hour at room temperature while stirring with a shaker. The solution in the wells was discarded, and each well was washed with HISCL washing solution. 100 µL of the HRP solution was added to each well, and the mixture was incubated for 1 hour at room temperature while stirring with a shaker. The solution in the wells was discarded, and each well was washed with HISCL washing solution. 100 µL of a substrate solution of HRP was added to each well, and chemiluminescence signal was measured with a plate reader. The samples were measured twice, and an average value of the two measured values was acquired. Results of measurement of the sample that contains the recombinant TTR are illustrated in Figs.14A to D. Results of the measurement of the blood sample are illustrated in Figs.15A and B.

Referring to Fig. 14 A, all of the signal measured values of the WT, T119M and V30M samples were higher than the signal measured value of the blank. This taught that the TTR tetramer can be detected by the detection method of this embodiment using the probe C1, the anti-TTR antibody immobilized on the solid phase, and the labeling substance. The signal measured value of T119M was higher than the signal measured value of WT, and the signal measured value of V30M was lower than the signal measured value of WT. Here, all of the samples containing the recombinant TTR were found to have a TTR level of 100 ng/mL, and the amount of each sample used for the measurement was the same (100 µL), suggesting that the graph of Fig.14A shows reactivity of each TTR tetramer with respect to the probe. That is, the results illustrated in Fig.14 A agreed that the stabilized mutant of TTR (T119M) formed a homotetramer having higher stability than that of wild-type TTR, and that the destabilized mutant of TTR (V30M) formed a homotetramer having lower stability than that of wild-type TTR. This taught that the stability of the TTR tetramer can be evaluated by the detection method of the present embodiment. Referring to Figs.14 B to D, results similar to those of Fig.14 A were also obtained when probes C2, C5, and C6 were used.

Referring to Fig. 15 A, the signal measured value of the serum sample increased according to the dilution factor. The regression line was expressed as y=140587x+1052.2 (R ² = 0.9974). Referring to Fig.15 B, the signal measured value of the plasma sample was similarly increased according to the dilution factor. The regression line was expressed as y=208070x+1139.4 (R ² = 0.9884). These results demonstrated that the TTR tetramer in the blood sample can be detected by the detection method of the present embodiment.

### EXAMPLE2: Detection of TTR tetramer in a sample (2)

The TTR tetramer in the sample containing the recombinant TTR and the blood sample was detected with use of the solid phase having the tagged probe immobilized thereon and the labeled anti-TTR antibody.

### (1) Sample and reagent

The sample containing the recombinant TTR and the blood sample were the same as in Example 1. Probes C5 and C6 were diluted with 1% BSA/0.5% casein/PBS, to prepare a probe solution (0.3 µM). An ALP-labeled polyclonal anti-TTR antibody is used as the labeled antibody capable of binding to the TTR monomer. The labeled antibody was prepared by labeling a polyclonal rabbit anti-human prealbumin antibody (Agilent, A0002) with ALP by a usual method. The ALP-labeled polyclonal anti-TTR antibody was diluted 6400 times with 1% BSA/0.1% goat IgG/0.01% mouse IgG/0.005% scavenger ALP/PBS, to prepare a labeled antibody solution. As a substrate solution of ALP, a mixed liquid (R4:R5=1:2) with HISCL R4 reagent (Sysmex Corporation) and HISCL R5 reagent (Sysmex Corporation) was used. HISCL R5 reagent was a reagent that contains CDP-star (trademark). As the solid phase, a black 96-well plate having streptavidin preliminarily immobilized on each well was used.

### (2) Detection of TTR tetramer

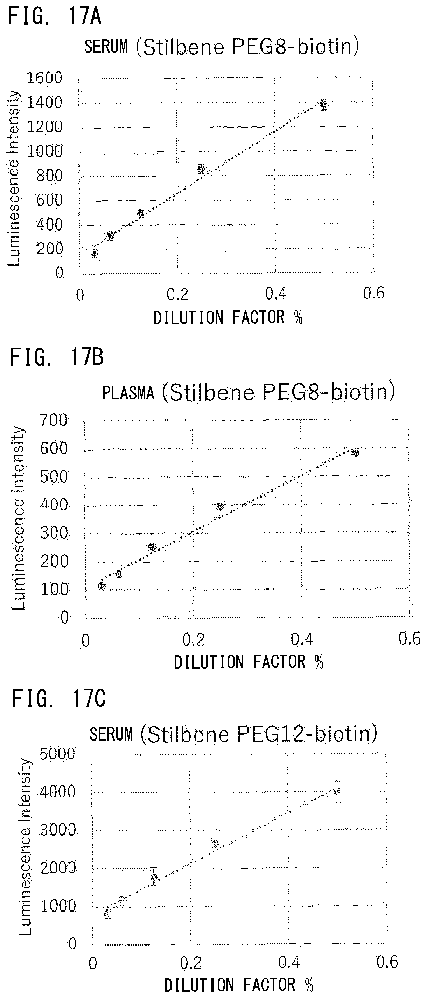

To each well of the plate, 300 µL of a blocking solution (1% BSA/0.5% casein/PBS) was added, followed by incubation for 1 hour at room temperature. The solution in the wells was discarded, and 100 µL of the probe solution was added separately, followed by incubation at room temperature for 0.5 hours with stirring with a shaker. The solution in the wells was discarded, and each well was washed with HISCL washing solution. The solution in the wells was discarded, and 100 µL of each sample was added to each well. The plate was incubated with stirring with a shaker at room temperature for 1 hour. As a control (blank), 100 µL of 1% BSA/0.5% casein/PBS was added to the wells, instead of the sample, and the wells were incubated in the same manner. The solution in the wells was discarded, and each well was washed with HISCL washing solution. 100 µL of the labeled antibody solution was added to each well, and the wells were incubated at room temperature for 1 hour with stirring with a shaker. The solution in the wells was discarded, and each well was washed with HISCL washing solution. 100 µL of a substrate solution of ALP was added to each well, and after 15 minutes, a chemiluminescence signal was measured with a plate reader. The samples were measured twice, and an average value of the two measured values was acquired. Results of measurement of the sample that contains the recombinant TTR are illustrated in Figs.16A and B. Measured results of the blood samples are illustrated in Figs. 17A to D.

Referring to Fig.16 A, all of the signal measured values of the WT, T119M and V30M samples were higher than the signal measured value of the blank. This taught that the TTR tetramer can be detected by the detection method of this embodiment that uses the probe C5 immobilized on the solid phase, and the labeled anti-TTR antibody. The signal measured value of T119M was higher than the signal measured value of WT, and the signal measured value of V30M was lower than the signal measured value of WT. This taught that the stability of the TTR tetramer can be evaluated by the detection method of the present embodiment. Referring to Fig.16 B, a result similar to that of Fig.16 A was also obtained when probe C6 was used.

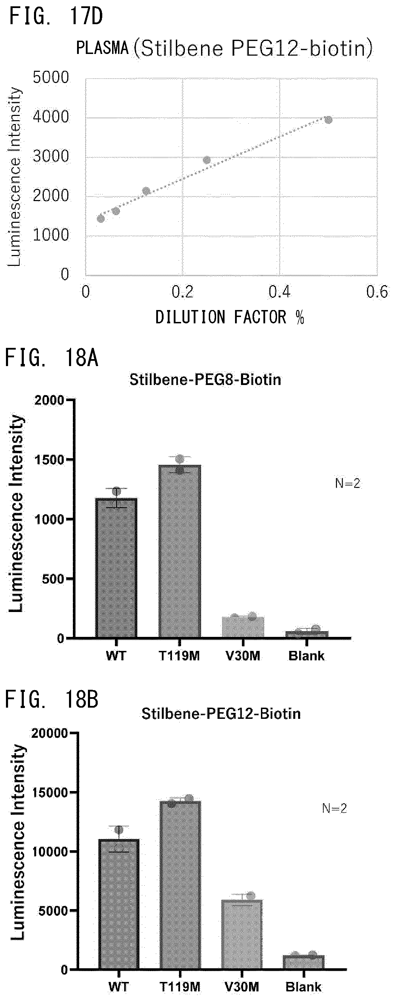

Referring to Fig.17 A, the signal measured value of the serum sample increased according to the dilution factor. The regression line was expressed as y=2528.6x+150.33 (R ² = 0.9884). Referring to Fig.17 B, the signal measured value of the plasma sample was similarly increased according to the dilution factor. The regression line was expressed as y=986.9x+108.19 (R ² = 0.9782). Referring to Fig.17 C, also in a case where Probe C6 immobilized on the solid phase was used, the signal measured value of the serum sample increased according to the dilution factor, similarly to the result of Fig.17 A. The regression line was expressed as y=6641.8x+793.35 (R ² = 0.9829). Referring to Fig.17D, the signal measured value of the plasma sample was similarly increased according to the dilution factor. The regression line was expressed as y=5353.2x+1382.4 (R ² = 0.9798). These results demonstrated that the TTR tetramer in the blood sample can be detected by the detection method of the present embodiment.

### EXAMPLE3: Detection of TTR tetramer in a sample (3)

The TTR tetramer in the sample containing the recombinant TTR and the blood sample was detected with use of the tagged probe, the labeled anti-TTR antibody, and the solid phase having the binding partner immobilized thereon.

### (1) Sample and reagent

The sample containing the recombinant TTR and the plasma sample were the same as in Example 1. Probes C5 and C6 were diluted with 1% BSA/0.5% casein/PBS, to prepare a probe solution (0.2 µM). The same ALP-labeled polyclonal anti-TTR antibody as in Example 2 was diluted 3200 times with 1% BSA/0.1% goat IgG/0.01% mouse IgG/0.005% scavenger ALP/PBS, to prepare a labeled antibody solution. The substrate solution and solid phase of ALP were the same as those in Example 2.

### (2) Detection of TTR tetramer

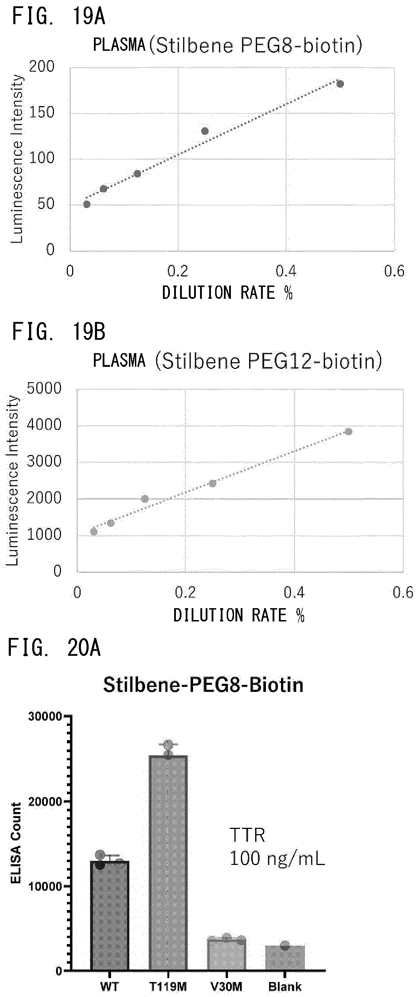

150 µL of each sample and 150 µL of the probe solution were mixed, and incubated at room temperature for 1 hour. As a control (blank), instead of the sample, 150 µL of 1% BSA/0.5% casein/PBS and 150 µL of the probe solution were mixed, and incubated in the same manner. 100 µL of the resultant mixed liquid was added to each well of the plate, and the resultant mixture was incubated for 1 hour at room temperature. The plate was then incubated with stirring with a shaker at room temperature for one hour. The solution in the wells was discarded, and each well was washed with HISCL washing solution. 100 µL of the labeled antibody solution was added to each well, and the wells were incubated at room temperature for 1 hour with stirring with a shaker. The solution in the wells was discarded, and each well was washed with HISCL washing solution. 100 µL of a substrate solution of ALP was added to each well, and after 15 minutes, a chemiluminescence signal was measured with a plate reader. The samples were measured twice, and an average value of the two measured values was acquired. Results of measurement of the sample that contains the recombinant TTR are illustrated in Figs.18A and B. Results of the measurement of the plasma samples are illustrated in Figs.19A and B.

Referring to Fig.18 A, all of the signal measured values of the WT, T119M and V30M samples were higher than the signal measured value of the blank. This taught that the TTR tetramer can be detected by the detection method of this embodiment using probe C5, the labeled anti-TTR antibody, and the solid phase. The signal measured value of T119M was higher than the signal measured value of WT, and the signal measured value of V30M was lower than the signal measured value of WT. This taught that the stability of the TTR tetramer can be evaluated by the detection method of the present embodiment. Referring to Fig.18 B, a result similar to that of Fig.18 A was also obtained when probe C6 was used.

Referring to Fig.19 A, the signal measured value of the plasma sample increased according to the dilution factor. The regression line was expressed as y=276.9x+49.25 (R ² = 0.9795). Referring to Fig.19 B, also in a case where the probe C6 was used, the signal measured value of the plasma sample increased according to the dilution factor, similarly to the result of Fig.19 A. The regression line was expressed as y=5631.7x+1050.8 (R ² = 0.9826). These results
This taught that the TTR tetramer in the blood sample can be detected by the detection method of the present embodiment.

### EXAMPLE4: Obtaining of a corrected value for the amount of TTR tetramer in the sample.

With use of probe C5, the TTR tetramer in the sample containing the recombinant TTR was detected in the same manner as in Example 1. Also, the total amount of recombinant TTR in the sample was measured by sandwich ELISA.

A corrected value regarding the amount of TTR tetramer was acquired based on the measured value obtained by the method of the present embodiment, and the measured value obtained by sandwich ELISA.

### (1) Sample and reagent

The sample containing the recombinant TTR was the same as in Example 1. Probe C5 was diluted with 1% BSA/0.5% casein/PBS to prepare a probe solution (10 µM). The anti-TTR antibody solution, the HRP solution, the substrate solution of HRP, and the solid phase were the same as those in Example 1. The labeled antibody solution and the substrate solution of ALP were the same as in Example 2.

### (2) Detection of TTR tetramer by the method of this embodiment.

TTR tetramer in each sample was detected in the same manner as in Example 1. Thereby, a measured value of the chemiluminescence signal was acquired as the first signal generated by the labeling substance (HRP) contained in the first complex. The samples were measured three times, and an average of three measured values was acquired. The result is illustrated in Fig.20 A.

### (3) Measurement of the total amount of TTR by sandwich ELISA

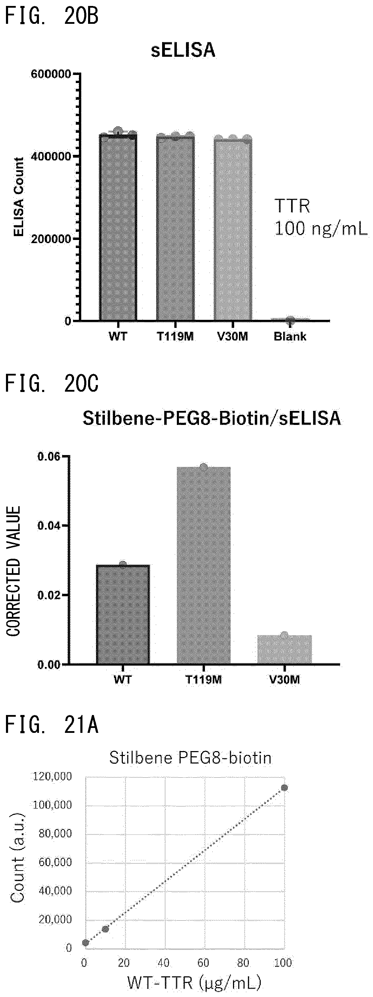

To each well of the plate, 100 µL of the anti-TTR antibody solution was added, followed by incubation at 4° C. overnight. The solution in the wells was discarded, and each well was washed with HISCL washing solution. To each well, 300 µL of a blocking solution (1% BSA/0.5% casein/PBS) was added, followed by incubation for 1 hour at room temperature. The solution in the wells was discarded, and 100 µL of each sample was added to each well. The plate was incubated with stirring with a shaker at room temperature for 1 hour. As a control (blank), 100 µL of 1% BSA/0.5% casein/PBS was added to the wells, instead of the sample, and the wells were incubated in the same manner. The solution in the wells was discarded, and each well was washed with HISCL washing solution. 100 µL of the labeled antibody solution was added to each well, and the wells were incubated at room temperature for 1 hour with stirring with a shaker. The solution in the wells was discarded, and each well was washed with HISCL washing solution. 100 µL of the HRP solution was added to each well, and the mixture was incubated for 1 hour at room temperature while stirring with a shaker. The solution in the wells was discarded, and each well was washed with HISCL washing solution. 100 µL of a substrate solution of ALP was added to each well, and after 15 minutes, a chemiluminescence signal was measured with a plate reader. Thereby, a measured value of the chemiluminescence signal was acquired as the second signal generated by the labeling substance (ALP) contained in the second complex. The samples were measured three times, and an average of three measured values was acquired. The result is illustrated in Fig.20 B.

### (4) Acquisition of a corrected value for the amount of TTR tetramer

A corrected value regarding the amount of TTR tetramer of each sample was acquired by dividing an average value of the measured values of the first signal by an average value of the measured values of the second signal. The result is illustrated in Fig.20C.

Referring to Fig.20 A, similarly to Example 1, the signal measured value of T119M was higher than the signal measured value of WT, and the signal measured value of V30M was lower than the signal measured value of WT. Referring to Fig.20 B, signal measured values of WT, T119M, and V30M acquired by sandwich ELISA were all comparable. This was consistent with that, in all samples, the TTR concentration was 100 ng/mL, and the amount of each sample used for the measurement was the same (100 µL). The corrected values of WT, T119M and V30M illustrated in Fig.20C therefore indicate the reactivity of each TTR tetramer per unit amount to the probe. The corrected value was proved to be useful for more accurate evaluation of the stability of TTR tetramer, because of the individual difference in TTR level in the blood.

### EXAMPLES: Detection of TTR tetramer with a fully automated immunoassay system (1)

The TTR tetramer in the sample containing the recombinant TTR was detected with use of a reagent that contains the tagged probe, a reagent that contains the labeled anti-TTR antibody, and a reagent that contains a solid phase having the binding partner immobilized thereon, with use of an fully automated immunoassay system HISCL-5000 (Sysmex Corporation).

### (1) sample

A sample containing wild-type (WT) recombinant TTR at respective concentrations of 10 µg/mL and 100 µg/mL was prepared by diluting transthyretin (wild-type) human, recombinant (AlexoTech, T-500-10) with 1% BSA/0.5% casein/PBS.

### (2) reagent

### (2.1) R1 reagent

As R1 buffer, a 0.1 mol/L HEPES buffer (pH 8.0±0.05) containing 1% BSA, 0.15 g/L mouse IgG, 0.1% sodium azide, 0.15 mol/L NaCl,0.1% Tween^{®} 20 and 20 mg/mL alkaline phosphatase (AP) mutein was prepared. Probe C5 was diluted with R1 buffer, to prepare R1 reagent (0.1 µM) containing probe C5. Probe C6 was also diluted with R1 buffer, to prepare R1 reagent (0.3 µM) that contains probe C6.

### (2.2) R3 reagent

As R3 buffer, a 0.1 mol/L HEPES buffer (pH 8.0±0.05) containing 1% BSA, 0.15 g/L murine IgG, 0.1% sodium azide, 0.15 mol/L NaCl,0.1% Tween20,20 mg/mL AP mutein, 1.0 mmol/L MgCl, and 0.1 mmol/L ZnCl of ₂ was prepared. An ALP-labeled monoclonal anti-TTR antibody was used as the labeled antibody capable of binding to the TTR monomer. The labeled antibody was prepared by labeling with ALP by a usual method, with a monoclonal mouse anti-human prealbumin antibody (Medix Biochemica Corporation, 100828, clone 11601). An ALP-labeled monoclonal anti-TTR antibody was diluted with R3 buffer to 0.5 pmol/L, to prepare an R3 reagent.

### (2.3) R2 Reagent, R4 Reagent and R5 Reagent

As the reagent that contains the solid phase, HISCL R2 reagent that contains streptavidin-bound magnetic particle (Sysmex Corporation) was used. HISCL R4 reagent (Sysmex Corporation) was used as the substrate buffer. HISCL R5 reagent (Sysmex Corporation) was used as the reagent that contains the substrate of ALP.

### (3) Detection of TTR tetramer

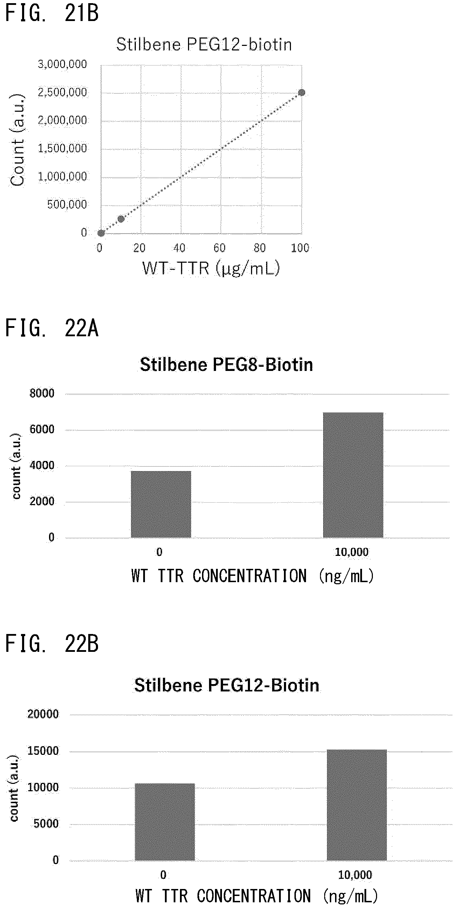

50 µL of each R1 reagent was added to the cuvette, to which 30 µL of the sample was then added, followed by incubation for 2.5 minutes. 30 µL of R2 reagent was added to the cuvette, followed by incubation for 1.5 minutes. The magnetic particle was magnetically collected to remove the supernatant, and the magnetic particle was washed with HISCL washing solution. The cleaning operation was conducted three times in total for two minutes. To the cuvette was added 100 µL of R3 reagent, followed by incubation for 2.5 minutes. The magnetic particle was magnetically collected to remove the supernatant, and the magnetic particle was washed with HISCL washing solution. The cleaning operation was conducted three times in total for two minutes. 50 µL of R4 reagent was added to the cuvette, and 100 µL of R5 reagent was added after 30 seconds. Five minutes later, the chemiluminescence signal was measured with HISCL-5000. As a control (blank), the sample was similarly measured with use of 30 µL of 1% BSA/0.5% casein/PBS instead of the sample. The results are illustrated in Figs.21A and B.

Referring to Fig.21A, the signal measured value increased corresponding to the level of recombinant TTR in the sample. The regression line was expressed as y=1090.1x+3673.1 (R ² = 0.9999). Referring to Fig.21B, also in a case where R1 reagent that contains probe C6 was used, the signal measured value increased corresponding to the level of the recombinant TTR in the sample, similarly to the result of Fig.21A. The regression line was expressed as y=25028x+7457 (R ² = 1). These results demonstrated that the TTR tetramer in the sample can be detected also by the detection method of the present embodiment using the fully automated immunoassay system.

### EXAMPLE6: Detection of TTR tetramer with a fully automated immunoassay system (2)

The TTR tetramer in the sample containing the recombinant TTR was detected with use of a reagent that contains the tagged probe, a labeling substance that contains the binding partner, and a reagent that contains a solid phase having an anti-TTR antibody immobilized thereon, with use of an fully automated immunoassay system HISCL-5000.

### (1) sample

A sample containing wild-type (WT) recombinant TTR at a concentration of 10 µg/mL was prepared by diluting transthyretin (wild-type) human, recombinant (AlexoTech, T-500-10) with 1% BSA/0.5% casein/PBS.

### (2) reagent

### (2.1) R1 reagent

As R1 buffer, a 0.1 mol/L HEPES buffer (pH 8.0±0.05) containing 1% BSA, 0.5% casein, 0.15 g/L mouse IgG, 0.1% sodium azide, 0.15 mol/L NaCl,0.1% Tween (registered trademark) 20 and 20 mg/mL AP mutein was prepared. In Example 6, R1 buffer was used as R1 reagent.

### (2.2) R2 reagent

A biotin-labeled monoclonal anti-TTR antibody was used as the labeled antibody capable of binding to the TTR monomer. The labeled antibody was prepared by labeling monoclonal mouse anti-human prealbumin antibody (Medix Biochemica, 100828, clone 11601) with biotin by a usual method. The biotin-labeled monoclonal anti-TTR antibody was diluted to 3 µg/mL with R1 buffer, to obtain an antibody solution. The antibody solution was mixed with the streptavidin-bound magnetic particle, to prepare an R2 reagent that contains the magnetic particle having the anti-TTR antibody immobilized thereon.

### (2.3) R3 reagent

As R3 buffer, a 0.1 mol/L HEPES buffer (pH 8.0±0.05) containing 1% BSA, 0.5% casein, 0.15 g/L murine IgG, 0.1% sodium azide, 0.15 mol/L NaCl,0.1% Tween20,20 mg/mL AP mutein, 1.0 mmol/L MgCl, and 0.1 mmol/L ZnCl of ₂ was prepared. Probes C5 and C6 were diluted with R3 buffer, to prepare R3 reagent (10 µM) containing each probe. Each R3 reagent was added at the time of use so as to adjust the final concentration of streptavidin-ALP (Promega Corporation, V5591, raw material Lot: 0000558623) to 1/1500.

### (2.4) R4 Reagent and R5 Reagent

HISCL R4 reagent (Sysmex Corporation) was used as the substrate buffer. HISCL R5 reagent (Sysmex Corporation) was used as the reagent that contains the substrate of ALP.

### (3) Detection of TTR tetramer

50 µL of R1 reagent was added to the cuvette, to which 30 µL of the sample was then added, followed by incubation for 2.5 minutes. 30 µL of R2 reagent was added to the cuvette, followed by incubation for 1.5 minutes. The magnetic particle was magnetically collected to remove the supernatant, and the magnetic particle was washed with HISCL washing solution. The cleaning operation was conducted three times in total for two minutes. To the cuvette, 100 µL of R3 reagent to which streptavidin-ALP was added was added, followed by incubation for 2.5 minutes. The magnetic particle was magnetically collected to remove the supernatant, and the magnetic particle was washed with HISCL washing solution. The cleaning operation was conducted three times in total for two minutes. 50 µL of R4 reagent was added to the cuvette, and 100 µL of R5 reagent was added after 30 seconds. Five minutes later, the chemiluminescence signal was measured with HISCL-5000. As a control (blank), the sample was similarly measured with use of 30 µL of 1% BSA/0.5% casein/PBS instead of the sample. The results are illustrated in Figs.22A and B.

Referring to Fig.22A, the signal measured value of the sample was higher than the signal measured value of the blank. Referring to Fig.22 B, a result similar to that of Fig.22 A was also obtained when probe C6 was used. These results demonstrated that the TTR tetramer in the sample can be detected also by the detection method of the present embodiment using the fully automated immunoassay system.

### Comparative Example 1: Detection of TTR by sandwich ELISA

TTR in the sample containing the recombinant TTR and the blood sample were detected by a usual sandwich ELISA using the capture antibody and the detection antibody.

### (1) Sample and calibrator

The recombinant TTR of the wild-type (WT), the stabilized variant (T119M), and the destabilized variant (V30M) as in Example 1 were used as the recombinant TTR. These recombinant TTRs were diluted with 1% BSA/0.5% casein/PBS, to prepare a sample containing 2.5 ng/mL of each recombinant TTR. Sera obtained from patients with atrial fibrillation (n = 26), wild-type ATTR patients (n = 18), and ATTR patients with destabilized mutation (A36D) (n = 2) were diluted with 1% BSA/PBS, to prepare various serum samples. A calibrator for preparing a calibration curve was prepared by serially diluting human plasma-derived prealbumin TTR (Athens Research and Technology, 16-16-161801) with 1% BSA/PBS.

### (2) reagent

### (2.1) A detection antibody and a labeling substance for measuring a sample containing the recombinant TTR.

An ALP-labeled polyclonal anti-TTR antibody was used as the detection antibody. The labeled antibody was prepared by labeling a polyclonal rabbit anti-human prealbumin antibody (Agilent, A0002) with ALP by a usual method. An ALP-labeled polyclonal anti-TTR antibody was diluted 6400 times with 1% BSA/0.1% goat IgG/0.01% mouse IgG/0.005% scavenger ALP/PBS, to prepare an ALP-labeled antibody solution.

### (2.2) Detection antibody for measuring serum sample, and labeling substance

A biotin-labeled polyclonal anti-TTR antibody was used as the detection antibody. The labeled antibody was prepared by labeling polyclonal rabbit anti-human prealbumin antibody (Agilent, A0002), with biotin by a usual method. The biotin-labeled monoclonal anti-TTR antibody was diluted to 0.5 µg/mL with 1% BSA/PBS, to obtain a biotin-labeled antibody solution. The labeling substance was streptavidin-labeled ALP (Vector, SA-5100-1). The streptavidin-labeled ALP was diluted 5000 times with 1% BSA/0.1% goat IgG/0.01% mouse IgG/0.005% scavenger ALP/PBS, to prepare an ALP solution.

### (2.3) Common reagent

A polyclonal rabbit anti-human prealbumin antibody (Agilent Inc., A0002) was used as the capture antibody. The antibody was diluted with PBS to prepare an anti-TTR antibody solution (2 µg/mL). As a substrate solution of ALP, a mixed liquid (R4:R5=1:2) with HISCL R4 reagent (Sysmex Corporation) and HISCL R5 reagent (Sysmex Corporation) was used. A black 96-well plate (Sumitomo Bakelite Corporation) was used as the solid phase.

### (3) Detection of TTR

### (3.1) Measurement of a Sample Containing Recombinant TTR

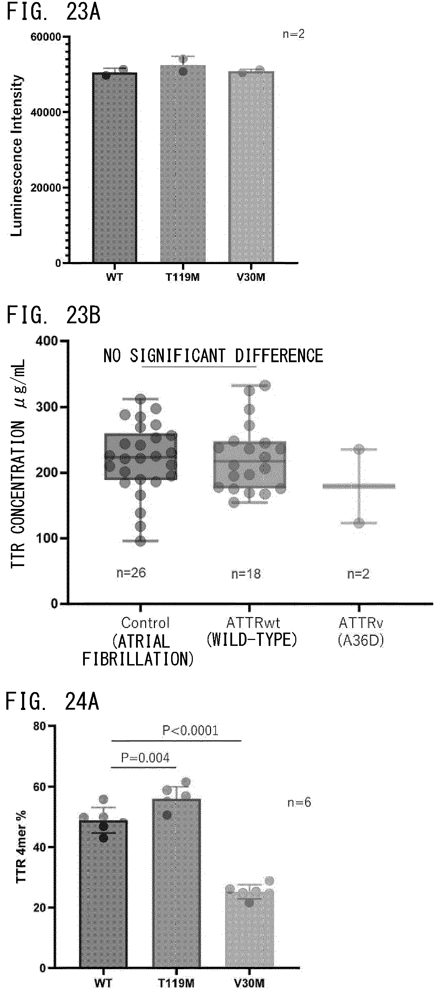

To each well of the plate, 100 µL of the anti-TTR antibody solution was added, followed by incubation at 4° C. overnight. The solution in the wells was discarded, and each well was washed with HISCL washing solution. To each well, 300 µL of a blocking solution (1% BSA/0.5% casein/PBS) was added, followed by incubation for 1 hour at room temperature. The solution in the wells was discarded, and 100 µL of each sample was added to each well. The plate was incubated with stirring with a shaker at room temperature for 1 hour. The solution in the wells was discarded, and each well was washed with HISCL washing solution. 100 µL of the ALP-labeled antibody solution was added to each well, and the wells were incubated at room temperature for 1 hour while stirring with a shaker. The solution in the wells was discarded, and each well was washed with HISCL washing solution. 100 µL of a substrate solution of ALP was added to each well, and after 15 minutes, a chemiluminescence signal was measured with a plate reader. The samples were measured twice, and an average value of the two measured values was acquired. The measured result is illustrated in Fig.23A.

### (3.2) Measurement of a serum sample

To each well of the plate, 100 µL of the anti-TTR antibody solution was added, followed by incubation at 4° C. overnight. The solution in the wells was discarded, and each well was washed with HISCL washing solution. To each well, 300 µL of a blocking solution (1% BSA/PBS) was added, followed by incubation for 1 hour at room temperature. The solution in the wells was discarded, and 100 µL of each blood sample was added to each well. The plate was incubated with stirring with a shaker at room temperature for 1 hour. The solution in the wells was discarded, and each well was washed with HISCL washing solution. 100 µL of the biotin-labeled antibody solution was added to each well, and the wells were incubated at room temperature for 1 hour with stirring with a shaker. The solution in the wells was discarded, and each well was washed with HISCL washing solution. 100 µL of the ALP solution was added to each well, and the mixture was incubated for 1 hour at room temperature while stirring with a shaker. The solution in the wells was discarded, and each well was washed with HISCL washing solution. 100 µL of a substrate solution of ALP was added to each well, and after 15 minutes, a chemiluminescence signal was measured with a plate reader. The calibrator was measured in the same manner. A calibration curve was prepared from the measured values of the calibrators, and the TTR level (µg/mL) of each blood sample was acquired with use of the calibration curve. The result is illustrated in Fig.23B.

Referring to Fig.23A, signal measured values of the samples of WT, T119M, and V30M were approximately the same. The capture antibody and the detection antibody used in the sandwich ELISA method can bind not only to the TTR tetramer but also to the TTR dimer and the TTR monomer, so that the signal measured value illustrated in Fig.23A is considered to indicate the total amount of TTR contained in each sample. This result indicated that the stability of TTR tetramer could not be evaluated by sandwich ELISA. Referring to Fig.23 B, there is no significant difference in TTR level between the group of patients with atrial fibrillation and the group of patients with wild-type ATTR. TTR levels in the ATTR patient group with the destabilizing mutation (A36D) appeared to be lower than those in the atrial fibrillation patient group and the wild-type ATTR patient group. However, since the number of patients was 2, there was no significant difference. This result indicated that ATTR could not be differentiated by sandwich ELISA.

### Comparative Example 2: Measurement of a 48-hour urea-treated sample by sandwich ELISA.

Referring to Patent Document 1, an unstable TTR that is not a tetramer was removed from the sample by urea treatment for 48 hours, and then the TTR tetramer in the sample was detected by sandwich ELISA.

### (1) Sample and calibrator

The recombinant TTR of the wild-type (WT), the stabilized variant (T119M), and the destabilized variant (V30M) as in Example 1 were used as the recombinant TTR. These recombinant TTRs were diluted with 1% BSA/PBS, to prepare a sample containing 16 µg/mL of each recombinant TTR. Sera obtained from patients with atrial fibrillation (n = 26), wild-type ATTR patients (n = 18), and ATTR patients with destabilized mutation (A36D) (n = 2) were diluted 10 times with 1% BSA/PBS, to prepare various serum samples. A calibrator was prepared in the same manner as in Comparative Example 1.

### (2) reagent

As the detection antibody and the labeling substance, a biotin-labeled antibody solution and an ALP solution were prepared in the same manner as in Comparative Example 1. An anti-TTR antibody solution was prepared as the capture antibody in the same manner as in Comparative Example 1. The substrate solution and the solid phase of ALP were the same as those in Comparative Example 1.

### (3) Urea treatment of the sample

A urea was added to the sample containing each recombinant TTR and each blood sample so as to have a final concentration of 7.2 M, and the sample was incubated at 25° C. for 48 hours. As a control, each sample was incubated without adding urea. After incubation, the sample containing the recombinant TTR was diluted with 1% BSA/PBS to a final concentration of 5 µg/mL, and the blood sample was diluted 6180 times with 1% BSA/PBS.

### (4) Detection of TTR tetramer

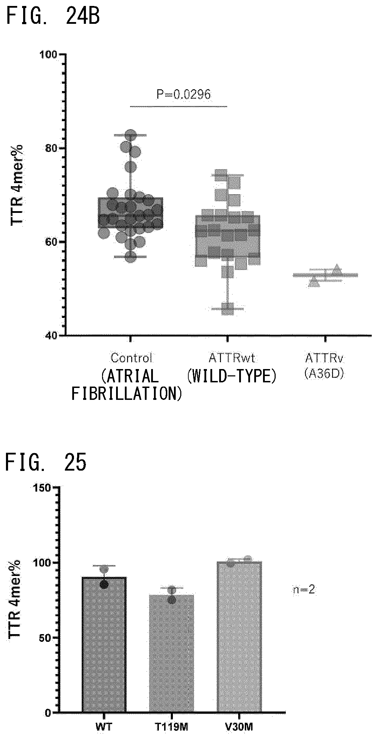

Each sample was measured by sandwich ELISA in the same manner as in (3.2) of Comparative Example 1. The calibrator was measured in the same manner. A calibration curve was prepared from the measured values of the calibrators, and the TTR level of each sample was acquired with use of the calibration curve. TTR 4 mer % of the sample after the urea treatment was calculated from the following calculation formula, using the TTR levels of the sample after the urea treatment and the sample without the urea treatment. TTR 4mer% showed the TTR tetramer level in the samples after the urea treatment. The significance test was performed by Dunnett's test. The results are illustrated in Figs.24A and B. 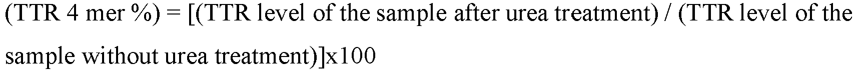 (TTR 4 mer %) = [(TTR level of the sample after urea treatment) / (TTR level of the sample without urea treatment)]x100

Referring to Fig.24 A, TTR 4mer% of the sample containing the mutant (T119M) TTR was significantly higher than that of the sample containing the wild-type TTR. Also, TTR 4mer% of mutant (V30M) TTR was significantly lower than that of the sample containing wild-type TTR. Referring to Fig.24 B, TTR 4mer% of the group of wild-type ATTR patients was significantly lower than that of the group of atrial fibrillation patients. TTR 4mer% of the destabilized mutation (A36D) in the ATTR patient group appeared to be lower than that of the atrial fibrillation patient group and the wild-type ATTR patient group. However, since the number of patients was 2, there was no significant difference. These results showed that the TTR tetramer in the sample can be detected by sandwich ELISA, when the sample that contains TTR is urea-treated for 48 hours. However, the sandwich ELISA method involving urea treatment of the sample cannot be said to be a simple detection method, since incubation for 48 hours or longer is required for urea treatment.

### Comparative Example 3: Measurement of a 1-hour urea-treated sample by sandwich ELISA.

We examined whether TTR tetramer could be detected by sandwich ELISA from the 1-hour urea-treated sample.

### (1) sample, calibrator, and reagent

The sample containing the recombinant TTR and the calibrator were the same as in Comparative Example 2. An ALP-labeled antibody solution was prepared as the detection antibody in the same manner as in Comparative Example 1. An anti-TTR antibody solution was prepared as the capture antibody in the same manner as in Comparative Example 1. The substrate solution and the solid phase of ALP were the same as those in Comparative Example 1.

### (2) Urea treatment of the sample

To the sample containing each recombinant TTR, urea was added to a final concentration of 7.2 M, followed by incubation at 25° C. for 1 hour. As a control, each sample was incubated without adding urea. After incubation, the sample containing the recombinant TTR was diluted with 1% BSA/PBS to a final concentration of 5 µg/mL.

### (3) Detection of TTR tetramer

Each sample was measured by sandwich ELISA in the same manner as in (3.1) of Comparative Example 1. The calibrator was measured in the same manner. A calibration curve was prepared from the measured values of the calibrators, and the TTR level of each sample was acquired with use of the calibration curve. TTR 4 mer % of the sample after the urea treatment was calculated from the calculation formula of Comparative Example 2, using the TTR levels of the sample after the urea treatment and the sample without the urea treatment. The result is illustrated in Fig.25.

Referring to Fig.25, there is no significant difference in TTR 4mer% of each sample of WT, T119M, and V30M. The TTR 4mer% of all the samples was 80% or higher, indicating that there is no large difference between the TTR level of the urea-treated sample and the TTR level of the untreated sample. That is, it was suggested that unstable TTR that is not a tetramer cannot be removed from the sample by the urea treatment for 1 hour. This result taught that the stability of the TTR tetramer cannot be evaluated by sandwich ELISA with 1 hour of urea treatment of the sample.

### [Explanation of letters or numerals]

10: Transthyretin (TTR) tetramer
11: Thyroxine (T4) binding site
20: A compound (ligand) capable of binding to the T4 binding site of the TTR tetramer
21: linker
22: Labeled probe
23: Tagged probe
30: An antibody capable of binding to the TTR monomer (anti-TTR antibody).
40: Solid phase
50: Labeling substance (enzyme)
60: Substrate for the enzyme
61: signal
70: tag
71: Binding partner that specifically binds to the tag
80: A kit comprising the reagent
81,91,101: first container
82,93,104: packing box
83,94,105: Attached Document
90,100: Reagent Kit
92,102: The second container
103: The third container

## Claims

1. A method for detecting transthyretin (TTR) tetramer in a sample, comprising:
forming a complex comprising the TTR tetramer in the sample, a compound capable of binding to the thyroxine binding site of the TTR tetramer, an antibody capable of binding to the TTR monomer, and a labeling substance; and
measuring a signal generated by the labeling substance contained in the complex.

2. The method according to claim 1, wherein the complex is formed on a solid phase in the forming step.

3. The method according to claim 2, wherein
the compound comprises a tag, and the labeling substance comprises a binding partner capable of specifically binding to the tag, and
in the forming step, the compound is labeled with the labeling substance by binding of the tag and the binding partner, and the antibody is immobilized on the solid phase.

4. The method according to claim 2, wherein
the antibody is labeled with the labeling substance,
the compound comprises a tag, and the solid phase comprises a binding partner that specifically binds to the tag, and
the compound is immobilized on the solid phase by binding of the tag and the binding partner in the forming step.

5. The method according to claim 2, wherein the compound is a compound immobilized on the solid phase, and the antibody is an antibody labeled with the labeling substance.

6. The method according to claim 2, wherein the compound is a compound labeled with the labeling substance, and
the antibody is immobilized on the solid phase in the forming step.

7. The method according to claim 1, wherein the labeling substance is an enzyme, a fluorescent substance, a compound that comprises a radioisotope, a chromogenic substance, or a chemiluminescent substance.

8. The method according to claim 1, wherein the compound is represented by formula (I): 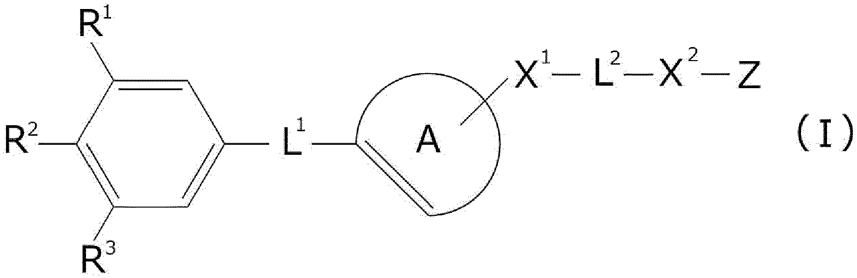
wherein R ¹ and R ³ are the same or different and each represents a halogen atom, a methyl group or a halogenated methyl group,
R ² represents a hydrogen-atom, a hydroxyl group or an amino-group,
L ¹ is a bond, an oxygen-atom, a sulfur-atom, or -CH=CH-, -CH ₂ -CH ₂ -, - N=N-, or - (C=O), 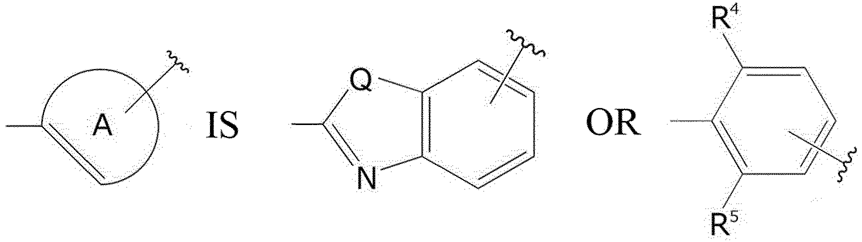
Q is an oxygen atom, a sulfur atom, or -NH-,
R ⁴ and R ⁵ are the same or different and represent a hydrogen atom or a halogen atom,
X ¹ and X ² are the same or different and are -R⁶-NH-, -NH-R⁶-, -R⁶-(C=O)-NH-, -(C=O)-NH-R⁶-, -R⁶-NH-(C=O)- , -E-(C=O)-R⁶-, -R⁶-(C=O)- , -(C=O)-R⁶-, -R⁶-(C=O)-O-, -(C=O)-O-R⁶-, -R⁶-O-(C=O)- , -O-(C=O)-R⁶-, -R⁶-(C=S)-NH-, -(C=S)-NH-R⁶-, -R⁶-NH-(C=S)- , -NH-(C=S)-R⁶-, -R⁶-O-, -O-R⁶-, -R⁶-S- or -S-R⁶,
R⁶ each independently represents a bond, an alkylene group having 1 to 10 carbon atoms which may have a substituent, an arylene group having 6 to 12 carbon atoms which may have a substituent, a heteroarylene group having 4 to 12 carbon atoms which may have a substituent, a cycloalkylene group having 3 to 8 carbon atoms which may have a substituent, or a heterocycloalkylene group having 2 to 8 carbon atoms which may have a substituent,
L ² is represented by -(CH ₂) ₐ -[X ³ - (CH ₂) _{b}]_{c} - or -[(CH ₂) _{b} - X ³] _{c} (CH ₂) ₐ - wherein X ³ is an oxygen atom, a sulfur atom, -NH-, -NH- (C = O) -, or a bond,
a and b are the same or different and are integers equal to or greater than 1 and equal to or less than 6,
c is an integer of 1 or more and 24 or less, and
Z comprises a tag, a labeling substance, or a solid phase.

9. The method according to claim 8, wherein the compound is represented by formula (II), formula (III) or formula (IV),
wherein the formula (II) is: 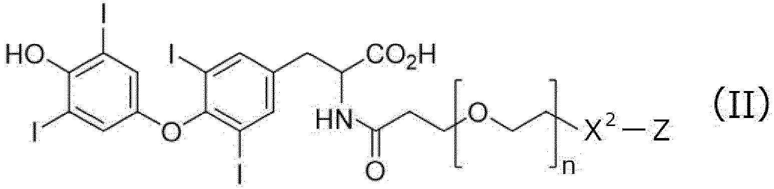
wherein Z comprises a labeling substance or a solid phase, and n is an integer of 1 or more and 24 or less,
X² is -R⁶-NH-, -NH-R⁶-, -R⁶-(C=O)-NH-, -(C=O)-NH-R⁶-, -R⁶-NH-(C=O)-, - NH-(C=O)-R⁶-, -R⁶-(C=O)-, -(C=O)-R⁶-, -R⁶-(C=O)-O-, -(C=O)-O-R⁶-, -R⁶-O-(C=O)-, -O-(C=O)-R⁶-, -R⁶-(C=S)-NH-, -(C=S)-NH-R⁶-, -R⁶-NH-(C=S)-, -NH-(C=S)-R⁶-, -R⁶-O-, -O-R⁶-, -R⁶-S- or -S-R⁶-,
R ⁶ each independently represents a bond, an alkylene group having 1 to 10 carbon atoms which may have a substituent, an arylene group having 6 to 12 carbon atoms which may have a substituent, a heteroarylene group having 4 to 12 carbon atoms which may have a substituent, a cycloalkylene group having 3 to 8 carbon atoms which may have a substituent, or a heterocycloalkylene group having 2 to 8 carbon atoms which may have a substituent, and
wherein the formula (III) is : 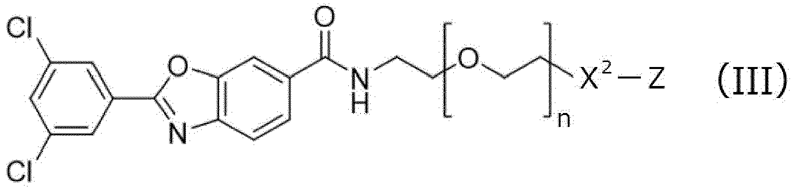
wherein Z comprises a labeling substance or a solid phase, and n is an integer of 1 or more and 24 or less,
X² is -R⁶-NH-, -NH-R⁶-, -R⁶-(C=O)-NH-, -(C=O)-NH-R⁶-, -R⁶-NH-(C=O)-, - NH-(C=O)-R⁶-, -R⁶-(C=O)-, -(C=O)-R⁶-, -R⁶-(C=O)-O-, -(C=O)-O-R⁶-, -R⁶-O-(C=O)-, -O-(C=O)-R⁶-, -R⁶-(C=S)-NH-, -(C=S)-NH-R⁶-, -R⁶-NH-(C=S)-, -NH-(C=S)-R⁶-, -R⁶-O-, -O-R⁶-, -R⁶-S- or -S-R⁶-,
R ⁶ each independently represents a bond, an alkylene group having 1 to 10 carbon atoms which may have a substituent, an arylene group having 6 to 12 carbon atoms which may have a substituent, a heteroarylene group having 4 to 12 carbon atoms which may have a substituent, a cycloalkylene group having 3 to 8 carbon atoms which may have a substituent, or a heterocycloalkylene group having 2 to 8 carbon atoms which may have a substituent,
wherein the formula (IV) is : 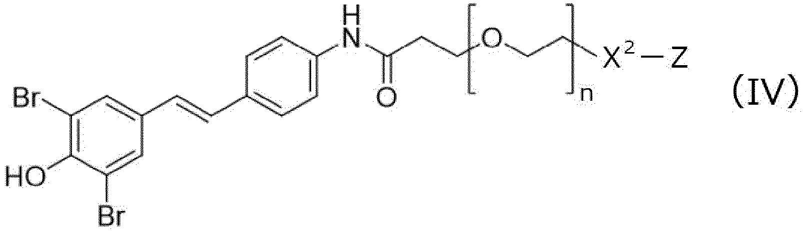
wherein Z comprises a labeling substance or a solid phase, and n is an integer of 1 or more and 24 or less,
X ² is -R⁶-NH-, -NH-R⁶-, -R⁶-(C=O)-NH-, -(C=O)-NH-R⁶-, -R⁶-NH-(C=O)-, - NH-(C=O)-R⁶-, -R⁶-(C=O)-, -(C=O)-R⁶-, -R⁶-(C=O)-O-, -(C=O)-O-R⁶-, -R⁶-O-(C=O)-, -O-(C=O)-R⁶-, -R⁶-(C=S)-NH-, -(C=S)-NH-R⁶-, -R⁶-NH-(C=S)-, -NH-(C=S)-R⁶-, -R⁶-O-, -O-R⁶-, -R⁶-S- o r-S-R⁶-,
R ⁶ each independently represents a bond, an alkylene group having 1 to 10 carbon atoms which may have a substituent, an arylene group having 6 to 12 carbon atoms which may have a substituent, a heteroarylene group having 4 to 12 carbon atoms which may have a substituent, a cycloalkylene group having 3 to 8 carbon atoms which may have a substituent, or a heterocycloalkylene group having 2 to 8 carbon atoms which may have a substituent.

10. The method according to claim 8, wherein the compound is represented by formula (V), formula (VI) or formula (VII),
wherein the formula (V) is 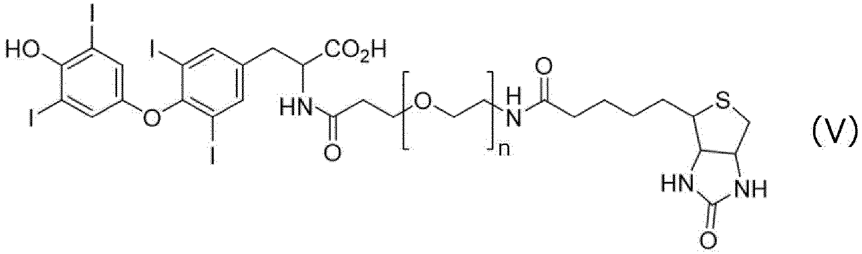
wherein n is an integer of 1 to 24,
wherein the formula (VI) is 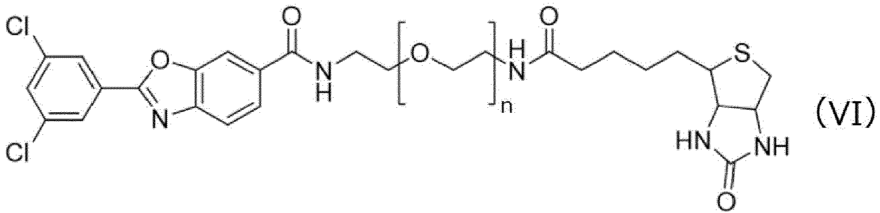
wherein n is an integer of 1 to 24,
wherein the formula (VII) is 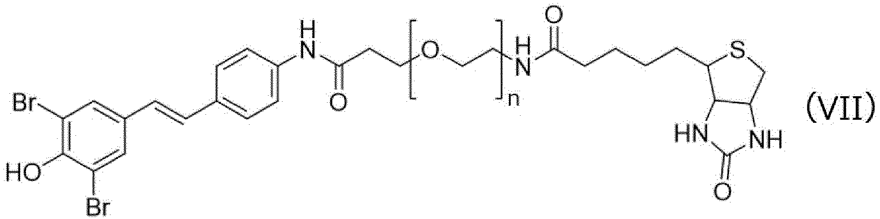
wherein n is an integer of 1 to 24.

11. A method for evaluating stability of transthyretin (TTR) tetramer in a sample, comprising:
forming a first complex comprising the TTR tetramer in the sample, a compound capable of binding to the thyroxine binding site of the TTR tetramer, an antibody capable of binding to the TTR monomer, and a labeling substance; and
measuring a first signal generated by the labeling substance in the first complex,
wherein the measured value of the first signal is indicative of the stability of the tetramer.

12. The method according to claim 11, further comprising:
forming a second complex comprising: the TTR monomer, TTR dimer or TTR tetramer in the sample; a capture antibody capable of binding to the TTR monomer; and a detection antibody that comprises a labeling substance and is capable of binding to the TTR monomer;
measuring a second signal generated by the labeling substance in the second complex; and
evaluating the stability of the TTR tetramer based on the measured value of the first signal and the measured value of the second signal.

13. The method according to claim 12, wherein in the evaluating step, a corrected value regarding an amount of the TTR tetramer is acquired from the measured value of the first signal and the measured value of the second signal, and the stability of the TTR tetramer is evaluated based on the corrected value.

14. The method according to claim 13, wherein the corrected value is a value acquired by dividing the measured value of the first signal by the measured value of the second signal.

15. The method according to claim 14, wherein
the TTR tetramer is evaluated to be stable if the corrected value is equal to or above a predetermined threshold value, and
the TTR tetramer is evaluated to be unstable if the corrected value is below a predetermine/d threshold value.

## Patentansprüche

1. Ein Verfahren zum Nachweis von Transthyretin (TTR)-Tetramer in einer Probe, umfassend:
Bilden eines Komplexes, der das TTR-Tetramer in der Probe, eine Verbindung, die in der Lage ist, an die Thyroxin-Bindungsstelle des TTR-Tetramers zu binden, einen Antikörper, der in der Lage ist, an das TTR-Monomer zu binden, und eine Markierungssubstanz umfasst; und
Messen eines Signals, das von der in dem Komplex enthaltenen Markierungssubstanz erzeugt wird.

2. Verfahren nach Anspruch 1, wobei der Komplex im Bildungsschritt an einer Festphase gebildet wird.

3. Verfahren nach Anspruch 2, wobei:
die Verbindung ein Tag umfasst und die Markierungssubstanz einen Bindungspartner umfasst, der in der Lage ist, spezifisch an das Tag zu binden, und
im Bildungsschritt die Verbindung durch Bindung des Tags und des Bindungspartners mit der Markierungssubstanz markiert wird und der Antikörper an der Festphase immobilisiert wird.

4. Verfahren nach Anspruch 2, wobei:
der Antikörper mit der Markierungssubstanz markiert ist,
die Verbindung ein Tag umfasst und die Festphase einen Bindungspartner umfasst, der spezifisch an das Tag bindet, und
die Verbindung im Bildungsschritt durch Bindung des Tags und des Bindungspartners an der Festphase immobilisiert wird.

5. Verfahren nach Anspruch 2, wobei die Verbindung eine an der Festphase immobilisierte Verbindung ist und der Antikörper ein mit der Markierungssubstanz markierter Antikörper ist.

6. Verfahren nach Anspruch 2, wobei die Verbindung eine mit der Markierungssubstanz markierte Verbindung ist, und
der Antikörper im Bildungsschritt an der Festphase immobilisiert wird.

7. Verfahren nach Anspruch 1, wobei die Markierungssubstanz ein Enzym, eine fluoreszierende Substanz, eine ein Radioisotop umfassende Verbindung, eine chromogene Substanz oder eine chemilumineszierende Substanz ist.

8. Verfahren nach Anspruch 1, wobei die Verbindung durch Formel (I) dargestellt wird: 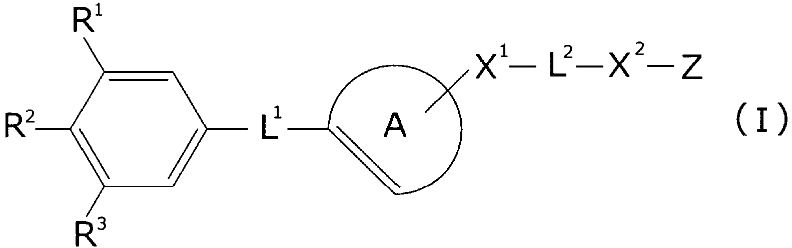
wobei R1 und R3 gleich oder verschieden sind und jeweils ein Halogenatom, eine Methylgruppe oder eine halogenierte Methylgruppe darstellen,
R2 ein Wasserstoffatom, eine Hydroxylgruppe oder eine Aminogruppe darstellt,
L1 eine Bindung, ein Sauerstoffatom, ein Schwefelatom oder -CH=CH-, -CH2-CH2-, -N=N- oder -(C=O) ist, 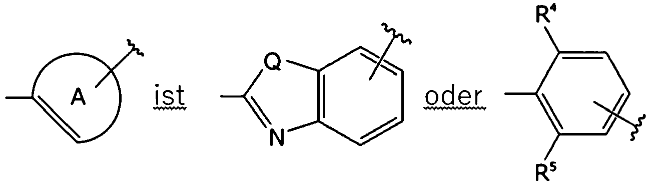
Q ein Sauerstoffatom, ein Schwefelatom oder -NH- ist,
R4 und R5 gleich oder verschieden sind und ein Wasserstoffatom oder ein Halogenatom darstellen,
X1 und X2 gleich oder verschieden sind und -R6-NH-, -NH-R6-, -R6-(C=O)-NH-, -(C=O)-NH-R6-, -R6-NH-(C=O)-, -NH-(C=O)-R6-, -R6-(C=O)-, -(C=O)-R6-, -R6-(C=O)-O-, - (C=O)-O-R6-, -R6-O-(C=O)-, -O-(C=O)-R6-, -R6-(C=S)-NH-, -(C=S)-NH-R6-, -R6-NH-(C=S)-, -NH-(C=S)-R6-, -R6-O-, -O-R6-, -R6-S- oder -S-R6- sind,
R6 jeweils unabhängig eine Bindung, eine Alkylengruppe mit 1 bis 10 Kohlenstoffatomen, die einen Substituenten aufweisen kann, eine Arylengruppe mit 6 bis 12 Kohlenstoffatomen, die einen Substituenten aufweisen kann, eine Heteroarylengruppe mit 4 bis 12 Kohlenstoffatomen, die einen Substituenten aufweisen kann, eine Cycloalkylengruppe mit 3 bis 8 Kohlenstoffatomen, die einen Substituenten aufweisen kann, oder eine Heterocycloalkylengruppe mit 2 bis 8 Kohlenstoffatomen, die einen Substituenten aufweisen kann, darstellt,
L2 durch -(CH2)a-[X3-(CH2)b]c- oder -[(CH2)b-X3]c(CH2)a- dargestellt wird, wobei X3 ein Sauerstoffatom, ein Schwefelatom, -NH-, -NH-(C=O)- oder eine Bindung ist,
a und b gleich oder verschieden sind und ganze Zahlen gleich oder größer als 1 und gleich oder kleiner als 6 sind,
c eine ganze Zahl von 1 oder mehr und 24 oder weniger ist, und Z ein Tag, eine Markierungssubstanz oder eine Festphase umfasst.

9. Verfahren nach Anspruch 8, wobei die Verbindung durch Formel (II), Formel (III) oder Formel (IV) dargestellt wird,
wobei die Formel (II) ist: 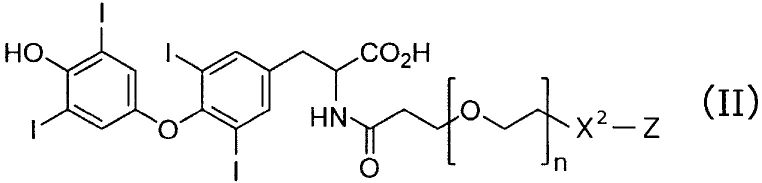
wobei Z eine Markierungssubstanz oder eine Festphase umfasst und n eine ganze Zahl von 1 oder mehr und 24 oder weniger ist,
X2 -R6-NH-, -NH-R6-, -R6-(C=O)-NH-, -(C=O)-NH-R6-, -R6-NH-(C=O)-, -NH-(C=O)-R6-, -R6-(C=O)-, -(C=O)-R6-, -R6-(C=O)-O-, -(C=O)-O-R6-, -R6-O-(C=O)-, - O-(C=O)-R6-, -R6-(C=S)-NH-, -(C=S)-NH-R6-, -R6-NH-(C=S)-, -NH-(C=S)-R6-, -R6-O-, -O-R6-, -R6-S- oder -S-R6- ist,
R6 jeweils unabhängig eine Bindung, eine Alkylengruppe mit 1 bis 10 Kohlenstoffatomen, die einen Substituenten aufweisen kann, eine Arylengruppe mit 6 bis 12 Kohlenstoffatomen, die einen Substituenten aufweisen kann, eine Heteroarylengruppe mit 4 bis 12 Kohlenstoffatomen, die einen Substituenten aufweisen kann, eine Cycloalkylengruppe mit 3 bis 8 Kohlenstoffatomen, die einen Substituenten aufweisen kann, oder eine Heterocycloalkylengruppe mit 2 bis 8 Kohlenstoffatomen, die einen Substituenten aufweisen kann, darstellt,
und wobei die Formel (III) ist: 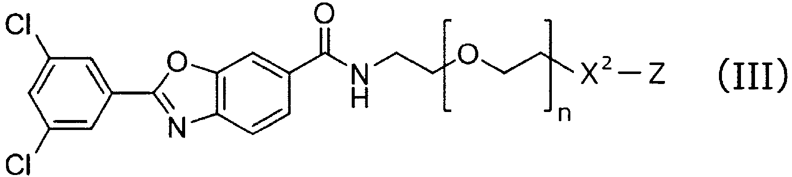
wobei Z eine Markierungssubstanz oder eine Festphase umfasst und n eine ganze Zahl von 1 oder mehr und 24 oder weniger ist,
X2 -R6-NH-, -NH-R6-, -R6-(C=O)-NH-, -(C=O)-NH-R6-, -R6-NH-(C=O)-, -NH-(C=O)-R6-, -R6-(C=O)-, -(C=O)-R6-, -R6-(C=O)-O-, -(C=O)-O-R6-, -R6-O-(C=O)-, - O-(C=O)-R6-, -R6-(C=S)-NH-, -(C=S)-NH-R6-, -R6-NH-(C=S)-, -NH-(C=S)-R6-, -R6-O-, -O-R6-, -R6-S- oder -S-R6- ist,
R6 jeweils unabhängig eine Bindung, eine Alkylengruppe mit 1 bis 10 Kohlenstoffatomen, die einen Substituenten aufweisen kann, eine Arylengruppe mit 6 bis 12 Kohlenstoffatomen, die einen Substituenten aufweisen kann, eine Heteroarylengruppe mit 4 bis 12 Kohlenstoffatomen, die einen Substituenten aufweisen kann, eine Cycloalkylengruppe mit 3 bis 8 Kohlenstoffatomen, die einen Substituenten aufweisen kann, oder eine Heterocycloalkylengruppe mit 2 bis 8 Kohlenstoffatomen, die einen Substituenten aufweisen kann, darstellt,
und wobei die Formel (IV) ist: 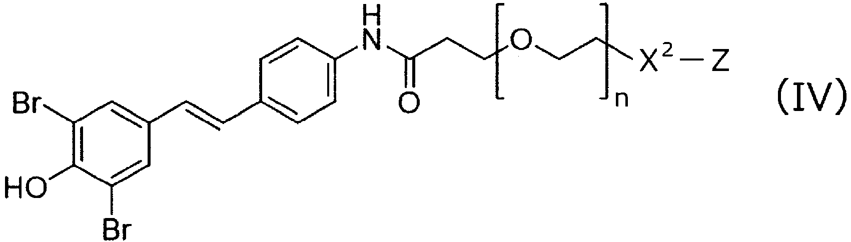
wobei Z eine Markierungssubstanz oder eine Festphase umfasst und n eine ganze Zahl von 1 oder mehr und 24 oder weniger ist,
X2 -R6-NH-, -NH-R6-, -R6-(C=O)-NH-, -(C=O)-NH-R6-, -R6-NH-(C=O)-, -NH-(C=O)-R6-, -R6-(C=O)-, -(C=O)-R6-, -R6-(C=O)-O-, -(C=O)-O-R6-, -R6-O-(C=O)-, - O-(C=O)-R6-, -R6-(C=S)-NH-, -(C=S)-NH-R6-, -R6-NH-(C=S)-, -NH-(C=S)-R6-, -R6-O-, -O-R6-, -R6-S- oder -S-R6- ist,
R6 jeweils unabhängig eine Bindung, eine Alkylengruppe mit 1 bis 10 Kohlenstoffatomen, die einen Substituenten aufweisen kann, eine Arylengruppe mit 6 bis 12 Kohlenstoffatomen, die einen Substituenten aufweisen kann, eine Heteroarylengruppe mit 4 bis 12 Kohlenstoffatomen, die einen Substituenten aufweisen kann, eine Cycloalkylengruppe mit 3 bis 8 Kohlenstoffatomen, die einen Substituenten aufweisen kann, oder eine Heterocycloalkylengruppe mit 2 bis 8 Kohlenstoffatomen, die einen Substituenten aufweisen kann, darstellt.

10. Verfahren nach Anspruch 8, wobei die Verbindung durch Formel (V), Formel (VI) oder Formel (VII) dargestellt wird,
wobei die Formel (V) ist: 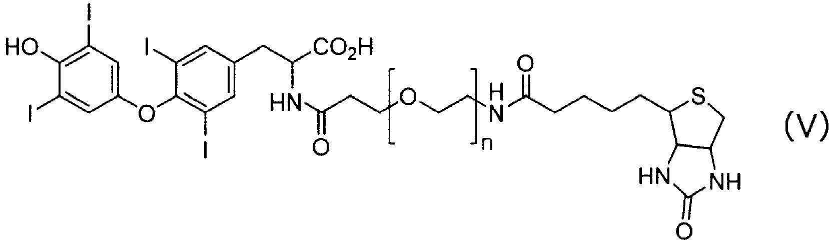
wobei n eine ganze Zahl von 1 bis 24 ist,
wobei die Formel (VI) ist: 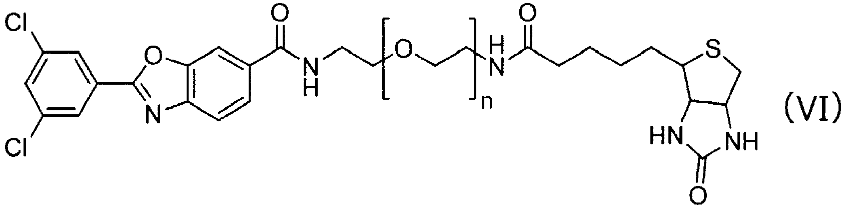
wobei n eine ganze Zahl von 1 bis 24 ist,
wobei die Formel (VII) ist: 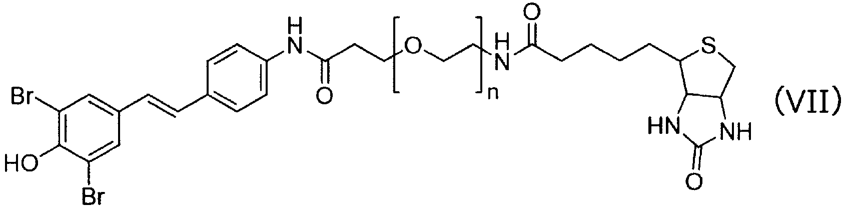
wobei n eine ganze Zahl von 1 bis 24 ist.

11. Ein Verfahren zur Bewertung der Stabilität von Transthyretin (TTR)-Tetramer in einer Probe, umfassend:
Bilden eines ersten Komplexes, der das TTR-Tetramer in der Probe, eine Verbindung, die in der Lage ist, an die Thyroxin-Bindungsstelle des TTR-Tetramers zu binden, einen Antikörper, der in der Lage ist, an das TTR-Monomer zu binden, und eine Markierungssubstanz umfasst; und
Messen eines ersten Signals, das von der Markierungssubstanz im ersten Komplex erzeugt wird,
wobei der gemessene Wert des ersten Signals indikativ für die Stabilität des Tetramers ist.

12. Verfahren nach Anspruch 11, ferner umfassend:
Bilden eines zweiten Komplexes, umfassend: das TTR-Monomer, TTR-Dimer oder TTR-Tetramer in der Probe; einen Fänger-Antikörper, der in der Lage ist, an das TTR-Monomer zu binden; und einen Detektions-Antikörper, der eine Markierungssubstanz umfasst und in der Lage ist, an das TTR-Monomer zu binden;
Messen eines zweiten Signals, das von der Markierungssubstanz im zweiten Komplex erzeugt wird; und
Bewerten der Stabilität des TTR-Tetramers basierend auf dem gemessenen Wert des ersten Signals und dem gemessenen Wert des zweiten Signals.

13. Verfahren nach Anspruch 12, wobei im Bewertungsschritt ein korrigierter Wert hinsichtlich einer Menge des TTR-Tetramers aus dem gemessenen Wert des ersten Signals und dem gemessenen Wert des zweiten Signals ermittelt wird und die Stabilität des TTR-Tetramers basierend auf dem korrigierten Wert bewertet wird.

14. Verfahren nach Anspruch 13, wobei der korrigierte Wert ein Wert ist, der durch Dividieren des gemessenen Werts des ersten Signals durch den gemessenen Wert des zweiten Signals ermittelt wird.

15. Verfahren nach Anspruch 14, wobei das TTR-Tetramer als stabil bewertet wird, wenn der korrigierte Wert gleich oder größer als ein vorbestimmter Schwellenwert ist, und das TTR-Tetramer als instabil bewertet wird, wenn der korrigierte Wert unter einem vorbestimmten Schwellenwert liegt.

## Revendications

1. Un procédé de détection du tétramère de la transthyrétine (TTR) dans un échantillon, comprenant :
• la formation d'un complexe comprenant le tétramère de TTR dans l'échantillon, un composé capable de se lier au site de liaison de la thyroxine du tétramère de TTR, un anticorps capable de se lier au monomère de TTR, et une substance de marquage ; et
• la mesure d'un signal généré par la substance de marquage contenue dans le complexe.

2. Procédé selon la revendication 1, dans lequel le complexe est formé sur une phase solide lors de l'étape de formation.

3. Procédé selon la revendication 2, dans lequel :
• le composé comprend une étiquette (tag), et la substance de marquage comprend un partenaire de liaison capable de se lier spécifiquement à l'étiquette, et
• lors de l'étape de formation, le composé est marqué avec la substance de marquage par la liaison de l'étiquette et du partenaire de liaison, et l'anticorps est immobilisé sur la phase solide.

4. Procédé selon la revendication 2, dans lequel :
• l'anticorps est marqué avec la substance de marquage,
• le composé comprend une étiquette, et la phase solide comprend un partenaire de liaison qui se lie spécifiquement à l'étiquette, et
• le composé est immobilisé sur la phase solide par la liaison de l'étiquette et du partenaire de liaison lors de l'étape de formation.

5. Procédé selon la revendication 2, dans lequel le composé est un composé immobilisé sur la phase solide, et l'anticorps est un anticorps marqué avec la substance de marquage.

6. Procédé selon la revendication 2, dans lequel le composé est un composé marqué avec la substance de marquage, et
• l'anticorps est immobilisé sur la phase solide lors de l'étape de formation.

7. Procédé selon la revendication 1, dans lequel la substance de marquage est une enzyme, une substance fluorescente, un composé qui comprend un radio-isotope, une substance chromogène, ou une substance chimiluminescente.

8. Procédé selon la revendication 1, dans lequel le composé est représenté par la formule (I) : 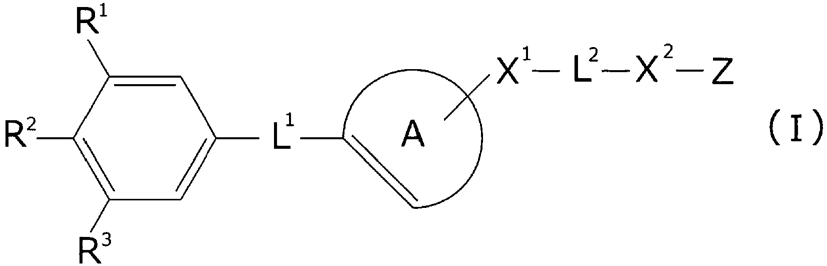
• dans laquelle R1 et R3 sont identiques ou différents et représentent chacun un atome d'halogène, un groupe méthyle ou un groupe méthyle halogéné,
• R2 représente un atome d'hydrogène, un groupe hydroxyle ou un groupe amino,
• L1 est une liaison, un atome d'oxygène, un atome de soufre, ou -CH=CH-, -CH2-CH2-, -N=N-, ou -(C=O),
• 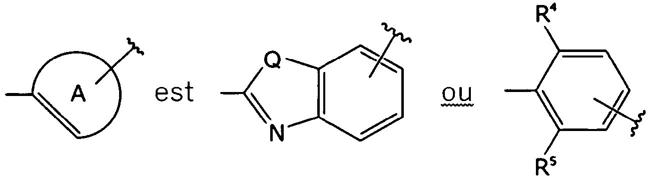
• Q est un atome d'oxygène, un atome de soufre, ou -NH-,
• R4 et R5 sont identiques ou différents et représentent un atome d'hydrogène ou un atome d'halogène,
• X1 et X2 sont identiques ou différents et sont -R6-NH-, -NH-R6-, -R6-(C=O)-NH-, -(C=O)-NH-R6-, -R6-NH-(C=O)-, -NH-(C=O)-R6-, -R6-(C=O)-, -(C=O)-R6-, - R6-(C=O)-O-, -(C=O)-O-R6-, -R6-O-(C=O)-, -O-(C=O)-R6-, -R6-(C=S)-NH-, - (C=S)-NH-R6-, -R6-NH-(C=S)-, -NH-(C=S)-R6-, -R6-O-, -O-R6-, -R6-S- ou -S-R6-,
• R6 représente chacun indépendamment une liaison, un groupe alkylène ayant 1 à 10 atomes de carbone qui peut avoir un substituant, un groupe arylène ayant 6 à 12 atomes de carbone qui peut avoir un substituant, un groupe hétéroarylène ayant 4 à 12 atomes de carbone qui peut avoir un substituant, un groupe cycloalkylène ayant 3 à 8 atomes de carbone qui peut avoir un substituant, ou un groupe hétérocycloalkylène ayant 2 à 8 atomes de carbone qui peut avoir un substituant,
• L2 est représenté par -(CH2)a-[X3-(CH2)b]c- ou -[(CH2)b-X3]c(CH2)a- où X3 est un atome d'oxygène, un atome de soufre, -NH-, -NH-(C=O)-, ou une liaison,
• a et b sont identiques ou différents et sont des entiers égaux ou supérieurs à 1 et égaux ou inférieurs à 6,
• c est un entier de 1 ou plus et 24 ou moins, et Z comprend une étiquette, une substance de marquage, ou une phase solide.

9. Procédé selon la revendication 8, dans lequel le composé est représenté par la formule (II), la formule (III) ou la formule (IV),
où la formule (II) est : 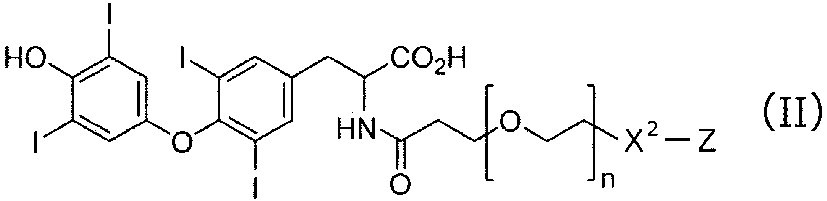
• dans laquelle Z comprend une substance de marquage ou une phase solide, et n est un entier de 1 ou plus et 24 ou moins,
• X2 est -R6-NH-, -NH-R6-, -R6-(C=O)-NH-, -(C=O)-NH-R6-, -R6-NH-(C=O)-, - NH-(C=O)-R6-, -R6-(C=O)-, -(C=O)-R6-, -R6-(C=O)-O-, -(C=O)-O-R6-, -R6-O-(C=O)-, -O-(C=O)-R6-, -R6-(C=S)-NH-, -(C=S)-NH-R6-, -R6-NH-(C=S)-, - NH-(C=S)-R6-, -R6-O-, -O-R6-, -R6-S- ou -S-R6-,
• R6 représente chacun indépendamment une liaison, un groupe alkylène ayant 1 à 10 atomes de carbone qui peut avoir un substituant, un groupe arylène ayant 6 à 12 atomes de carbone qui peut avoir un substituant, un groupe hétéroarylène ayant 4 à 12 atomes de carbone qui peut avoir un substituant, un groupe cycloalkylène ayant 3 à 8 atomes de carbone qui peut avoir un substituant, ou un groupe hétérocycloalkylène ayant 2 à 8 atomes de carbone qui peut avoir un substituant,
et où la formule (III) est : 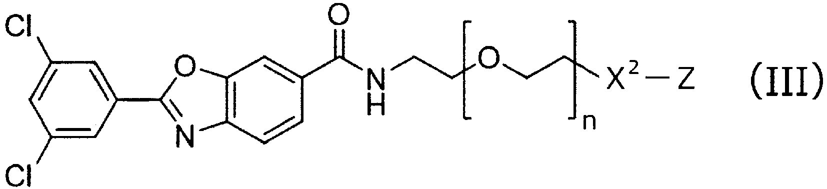
• dans laquelle Z comprend une substance de marquage ou une phase solide, et n est un entier de 1 ou plus et 24 ou moins,
• X2 est -R6-NH-, -NH-R6-, -R6-(C=O)-NH-, -(C=O)-NH-R6-, -R6-NH-(C=O)-, - NH-(C=O)-R6-, -R6-(C=O)-, -(C=O)-R6-, -R6-(C=O)-O-, -(C=O)-O-R6-, -R6-O-(C=O)-, -O-(C=O)-R6-, -R6-(C=S)-NH-, -(C=S)-NH-R6-, -R6-NH-(C=S)-, - NH-(C=S)-R6-, -R6-O-, -O-R6-, -R6-S- ou -S-R6-,
• R6 représente chacun indépendamment une liaison, un groupe alkylène ayant 1 à 10 atomes de carbone qui peut avoir un substituant, un groupe arylène ayant 6 à 12 atomes de carbone qui peut avoir un substituant, un groupe hétéroarylène ayant 4 à 12 atomes de carbone qui peut avoir un substituant, un groupe cycloalkylène ayant 3 à 8 atomes de carbone qui peut avoir un substituant, ou un groupe hétérocycloalkylène ayant 2 à 8 atomes de carbone qui peut avoir un substituant,
et où la formule (IV) est : 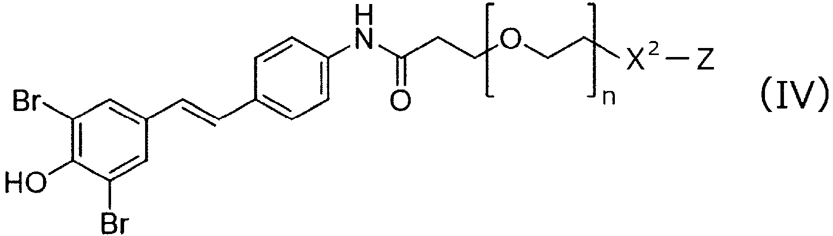
dans laquelle Z comprend une substance de marquage ou une phase solide, et n est un entier de 1 ou plus et 24 ou moins,
• X2 est -R6-NH-, -NH-R6-, -R6-(C=O)-NH-, -(C=O)-NH-R6-, -R6-NH-(C=O)-, - NH-(C=O)-R6-, -R6-(C=O)-, -(C=O)-R6-, -R6-(C=O)-O-, -(C=O)-O-R6-, -R6-O-(C=O)-, -O-(C=O)-R6-, -R6-(C=S)-NH-, -(C=S)-NH-R6-, -R6-NH-(C=S)-, - NH-(C=S)-R6-, -R6-O-, -O-R6-, -R6-S- ou -S-R6-,
• R6 représente chacun indépendamment une liaison, un groupe alkylène ayant 1 à 10 atomes de carbone qui peut avoir un substituant, un groupe arylène ayant 6 à 12 atomes de carbone qui peut avoir un substituant, un groupe hétéroarylène ayant 4 à 12 atomes de carbone qui peut avoir un substituant, un groupe cycloalkylène ayant 3 à 8 atomes de carbone qui peut avoir un substituant, ou un groupe hétérocycloalkylène ayant 2 à 8 atomes de carbone qui peut avoir un substituant.

10. Procédé selon la revendication 8, dans lequel le composé est représenté par la formule (V), la formule (VI) ou la formule (VII),
où la formule (V) est : 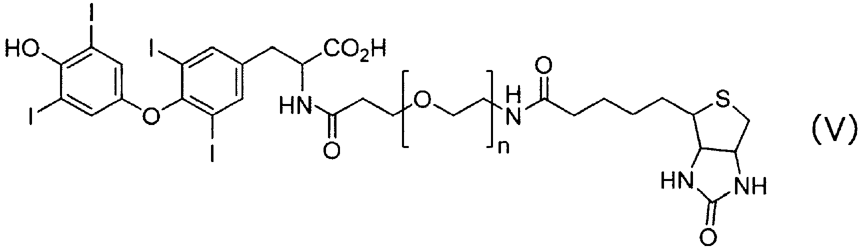
• dans laquelle n est un entier de 1 à 24,
où la formule (VI) est : 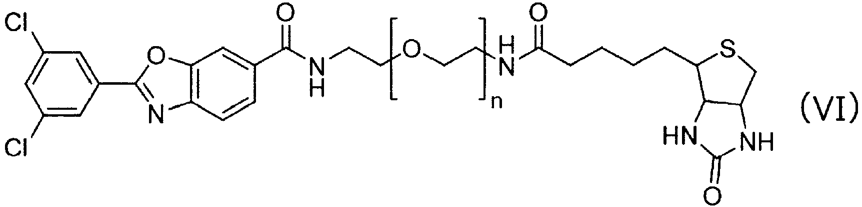
• dans laquelle n est un entier de 1 à 24,
où la formule (VII) est : 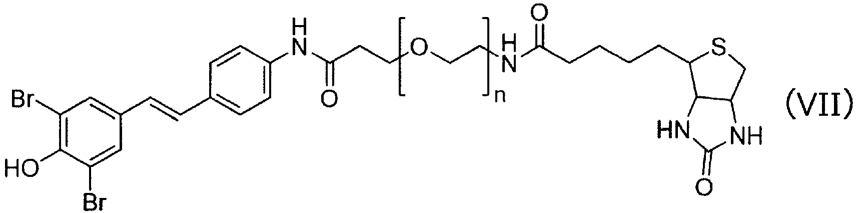
• dans laquelle n est un entier de 1 à 24.

11. Un procédé d'évaluation de la stabilité du tétramère de la transthyrétine (TTR) dans un échantillon, comprenant :
• la formation d'un premier complexe comprenant le tétramère de TTR dans l'échantillon, un composé capable de se lier au site de liaison de la thyroxine du tétramère de TTR, un anticorps capable de se lier au monomère de TTR, et une substance de marquage ; et
• la mesure d'un premier signal généré par la substance de marquage dans le premier complexe,
• dans lequel la valeur mesurée du premier signal est indicative de la stabilité du tétramère.

12. Procédé selon la revendication 11, comprenant en outre :
• la formation d'un second complexe comprenant : le monomère de TTR, le dimère de TTR ou le tétramère de TTR dans l'échantillon ; un anticorps de capture capable de se lier au monomère de TTR ; et un anticorps de détection qui comprend une substance de marquage et est capable de se lier au monomère de TTR ;
• la mesure d'un second signal généré par la substance de marquage dans le second complexe ; et
• l'évaluation de la stabilité du tétramère de TTR sur la base de la valeur mesurée du premier signal et de la valeur mesurée du second signal.

13. Procédé selon la revendication 12, dans lequel, lors de l'étape d'évaluation, une valeur corrigée concernant une quantité du tétramère de TTR est acquise à partir de la valeur mesurée du premier signal et de la valeur mesurée du second signal, et la stabilité du tétramère de TTR est évaluée sur la base de la valeur corrigée.

14. Procédé selon la revendication 13, dans lequel la valeur corrigée est une valeur acquise en divisant la valeur mesurée du premier signal par la valeur mesurée du second signal.

15. Procédé selon la revendication 14, dans lequel le tétramère de TTR est évalué comme étant stable si la valeur corrigée est égale ou supérieure à une valeur seuil prédéterminée, et le tétramère de TTR est évalué comme étant instable si la valeur corrigée est inférieure à une valeur seuil prédéterminée.
